# EUROPÄISCHE PATENTSCHRIFT

(11) **EP 3 995 137 B1**
(45) Veröffentlichungstag und Bekanntmachung des Hinweises auf die Patenterteilung: **03.01.2024**
(21) Anmeldenummer: 21206774.8
(22) Anmeldetag: 05.11.2021
(51) Int. Cl.: A61K 9/70, A61K 31/135, A61K 47/34, A61P 29/00

(54) **ORALER DÜNNFILM**
ORAL THIN FILM
FILM MINCE ORALE

(30) Priorität: 09.11.2020 DE 102020129394; 11.08.2021 DE 102021120937
(43) Veröffentlichungstag der Anmeldung: 11.05.2022
(73) Patentinhaber: LTS Lohmann Therapie-Systeme AG, 56626 Andernach (DE)
(72) Erfinder: MÜLLER, Markus, 53840 Troisdorf (DE); FICKER, Mario, 53179 Bonn (DE); LINN, Michael, 55596 Waldböckelheim (DE); HAMMES, Florian, 56626 Andernach (DE)
(74) Vertreter: Meissner Bolte Partnerschaft mbB

(56) Entgegenhaltungen:
- WO-A1-2019/110727
- CA-A1- 3 131 148
- US-A1- 2014 079 740
- US-A1- 2015 342 947
- US-A1- 2020 138 714

## Beschreibung

Die vorliegende Erfindung betrifft einen oralen Dünnfilm enthaltend mindestens einen pharmazeutisch aktiven Wirkstoff, ein Verfahren zu dessen Herstellung, sowie die Verwendung eines solchen oralen Dünnfilms als Arzneimittel, insbesondere in der Behandlung von Schmerzen und/oder Depressionen.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne wirkstoffhaltige Filme auf Polymerbasis, die, wenn sie auf eine Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Die US 2020/138714A1 offenbart einen schnell zerfallenden Schaumwafer mit hohem Flächengewicht.

Wie nachstehend näher erläutert, enthält der erfindungsgemäße orale Dünnfilm vorzugsweise Ketamin als pharmazeutisch aktiven Wirkstoff und dient bevorzugt der Behandlung oder Prävention von Schmerzen.

Die medikamentöse Neupositionierung von Ketamin als Analgetikum deutet auf die Möglichkeit einer neuen Schmerzbehandlungsoption hin. Ketamin hat sich bei der Behandlung von mäßigen bis starken Schmerzen als wirksam erwiesen und stellt eine nützliche Alternative zur Opioid-Analgesie dar. Ketamin reduziert nachweislich auch die Hyperalgesie (die erhöhte Schmerzempfindlichkeit), die bei vielen Schmerzzuständen auftritt, und zwar auch durch die langfristige Einnahme von Opioiden. In Kombination mit Opioiden hat Ketamin auch eine dosisschonende Wirkung auf die Menge an Opioid, die zur Erreichung einer Analgesie erforderlich ist.

Der NMDA-Rezeptor-Antagonismus von Ketamin bietet eine "Nicht-Opioid"-Behandlungsoption für die Schmerzbehandlung, die die unerfüllten Bedürfnisse der gegenwärtigen Therapie (z.B. verringerte schwere Nebenwirkungen im Zusammenhang mit Opioiden) abdeckt. (S)-Ketamin hat im Vergleich zum Racemat eine in etwa doppelt so hohe analgetische, als auch antidepressive Wirkung. Im Gegensatz zu Opioiden ist die letale Dosis von (S)-Ketamin sehr hoch (letale Dosis gemittelt bei 4,2 g/70 kg; z.B. letale Dosis Fentanyl 2 mg/70 kg, Oxycodon 40 mg/70 kg).

Bei der Verabreichung von manchen pharmazeutisch aktiven Wirkstoffen sind hohe Wirkstoffbeladungen des oralen Dünnfilms wünschenswert. Eine hohe Wirkstoffbeladung in oralen Dünnfilmen ist ein bekanntes Problem, da diese zu brüchigen Filmen führen oder eine Filmbildung der enthaltenen Polymere direkt unterbinden kann. Um dies dennoch zu erreichen, werden in der Regel große orale Dünnfilme oder orale Dünnfilme mit sehr hohen Schichtdicken benötigt. Große oder dicke orale Dünnfilme haben den Nachteil, dass sie Probleme bei der Applikation verursachen und zu Fremdkörpergefühlen bei den Patienten sowie zu langen Auflösezeiten führen können.

Je nach Anwendung sind lange Zerfallszeiten jedoch unerwünscht.

Zudem haben bekannte orale Dünnfilme mit hoher Wirkstoffbeladung den Nachteil, dass das maximale Flächengewicht und damit die Menge an enthaltenem pharmazeutisch aktivem Wirkstoff durch die Trocknung des oralen Dünnfilms bei dessen Herstellung bestimmt wird. Je größer das Flächengewicht des oralen Dünnfilms ist, desto mehr pharmazeutisch aktiver Wirkstoff kann zwar darin enthalten sein, jedoch wird dadurch die Trockenzeit des oralen Dünnfilms auf eine nicht mehr wirtschaftliche Zeit verlängert und es kann zudem zu einer inhomogenen Verteilung des Wirkstoffs in dem oralen Dünnfilm kommen.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm zur Verabreichung einer hohen Menge an mindestens einem pharmazeutisch aktiven Wirkstoff bereitzustellen, wobei der orale Dünnfilm eine akzeptable Zerfallszeit aufweist, und wobei der pharmazeutisch aktive Wirkstoff relativ homogen in dem oralen Dünnfilm verteilt ist. Ferner soll der orale Dünnfilm eine möglichst angenehme und weiche Textur aufweisen und damit möglichst kein Fremdkörpergefühl bei den Patienten auslösen. Der erfindungsgemäße orale Dünnfilm soll ferner eine möglichst hohe Bioverfügbarkeit von beispielsweise mehr als 10 % oder mehr als 20 % oder mehr als 30 % oder mehr als 40 % oder mehr als 50 % oder mehr als 60% oder mehr als 70% oder mehr als 80 % oder mehr als 90 % des pharmazeutisch aktiven Wirkstoffs ermöglichen.

Vorzugsweise weist der erfindungsgemäße orale Dünnfilm durch eine Bioverfügbarkeit 20 bis 30% des pharmazeutisch aktiven Wirkstoffs auf.

Zudem soll der erfindungsgemäße orale Dünnfilm so gestaltet sein, dass innerhalb der ersten Minute nach Applikation etwa 40 bis 60 % des enthaltenen pharmazeutisch aktiven Wirkstoffs freigesetzt werden können bzw. nach den ersten zwei Minuten nach Applikation etwa 75 bis 90 % des enthaltenen pharmazeutisch aktiven Wirkstoffs freigesetzt werden können.

Außerdem sollen nach Einnahme des oralen Dünnfilms möglichst wenige Nebenwirkungen, insbesondere möglichst wenige psychedelische Effekte (psychische und psychomimetische Nebenwirkungen), auftreten.

Zudem soll der orale Dünnfilm möglichst einfach und wirtschaftlich herstellbar sein.

Insbesondere soll der mindestens eine pharmazeutisch aktive Wirkstoff Ketamin umfassen.

Obige Aufgabe wird durch einen oralen Dünnfilm nach Anspruch 1 gelöst, der mindestens eine Matrixschicht aufweist, wobei die mindestens eine Matrixschicht mindestens einen pharmazeutisch aktiven Wirkstoff, mindestens einen Polyvinylalkohol und mindestens ein Polyvinylalkohol-Polyethylenglycol-Pfropfcopolymer umfasst, wobei der mindestens eine pharmazeutisch aktive Wirkstoff Ketamin oder ein pharmazeutisch akzeptables Salz davon umfasst.

Es wurde herausgefunden, dass ein Polyvinylalkohol-Polyethylenglycol-Pfropfcopolymer einen hohen Anteil an Wirkstoff aufnehmen kann.

Dies wurde auch schon bei Polyvinylalkohol beobachtet, wo dieser Effekt teilweise noch ausgeprägter ist. Filme auf Basis von Polyvinylalkohol alleine sind jedoch hart und weisen damit eine für den Patienten unangenehme Textur auf.

Filme auf Basis von einem Polyvinylalkohol-Polyethylenglycol-Pfropfcopolymer alleine dagegen weisen eine weichere Textur auf, da das Polymer selbst weichere Filme bildet. Diese Filme sind aber nicht ganz so stabil.

Durch die Mischung aus einem Polyvinylalkohol-Polyethylenglycol-Pfropfcopolymer und einem Polyvinylalkohol konnte erreicht werden, dass der Film bei hoher Wirkstoffbeladung die weiche Textur eines Polyvinylalkohol-Polyethylenglycol-Pfropfcopolymer-Films bei annähernder Stabilität des Polyvinylalkohol-Films aufweist.

Das Polyvinylalkohol-Polyethylenglycol-Pfropfcopolymer bildet vorzugsweise das Grundgerüst und bestimmt maßgeblich die Filmeigenschaften und der Polyvinylalkohol wirkt als zusätzlicher Stabilisator.

So kann eine hohe Wirkstoffbeladung mit Schichtdicken und Filmgrößen in akzeptablen Bereichen erreicht werden. Auch die Auflösezeiten liegen im akzeptablen Bereich, wobei der akzeptable Bereich vorzugsweise Werte von weniger als 1 min umfasst. Zudem ist ein solcher oraler Dünnfilm angenehm für den Patienten und kann einfach und wirtschaftlich hergestellt werden.

Bevorzugte Ausführungsformen sind in den abhängigen Ansprüchen angegeben.

In der vorliegenden Schrift kann "umfassend" auch "bestehend aus" bedeuten.

Der erfindungsgemäße orale Dünnfilm weist mindestens eine Matrixschicht auf, wobei die mindestens eine Matrixschicht mindestens einen pharmazeutisch aktiven Wirkstoff, mindestens einen Polyvinylalkohol und mindestens ein Polyvinylalkohol-Polyethylenglycol-Pfropfcopolymer umfasst, wobei der mindestens eine pharmazeutisch aktive Wirkstoff Ketamin oder ein pharmazeutisch akzeptables Salz davon umfasst.

Der mindestens eine pharmazeutisch aktive Wirkstoff umfasst Ketamin oder ein pharmazeutisch akzeptables Salz davon.

Gemäß der vorliegenden Erfindung werden unter dem pharmazeutisch aktiven Wirkstoff auch alle pharmazeutisch akzeptablen Salze und Solvate des jeweiligen pharmazeutisch aktiven Wirkstoffs subsumiert.

Polyvinylalkohole (abgekürzt mit PVA oder PVAL, gelegentlich auch mit PVOH) sind Polymere der allgemeinen Struktur 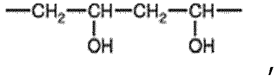 die in geringen Anteilen (etwa 2 %) auch Struktureinheiten des Typs 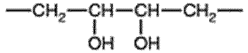 enthalten können. Sie gehören zur Gruppe der Vinylpolymere.

Handelsübliche Polyvinylalkohole, die als weiß-gelbliche Pulver oder Granulate angeboten werden, haben in der Regel Hydrolysegrade von 98 bis 99 bzw. 87 bis 89 Mol-%, enthalten also noch einen Restgehalt an Acetyl-Gruppen. Charakterisiert werden die Polyvinylalkohole von Seiten der Hersteller durch Angabe des Polymerisationsgrades des Ausgangspolymeren bzw. des mittleren Molekulargewichtes, des Hydrolysegrades, der Verseifungszahl bzw. der Lösungsviskosität.

Pfropfcopolymere sind verzweigte Polymere, die unterschiedliche Monomereinheiten in der Hauptkette und der verzweigten Kette enthalten.

Gebräuchlich ist für Pfropfcopolymere auch die englische Bezeichnung "graft copolymers".

Das hier vorliegende Polyvinylalkohol-Polyethylenglycol-Pfropfcopolymer weist vorzugsweise eine Hauptkette, umfassend Polyethylenglycol auf, auf die Polyvinylalkohol-Einheiten aufgepfropft sind.

Der erfindungsgemäße orale Dünnfilm ist ferner bevorzugt dadurch gekennzeichnet, dass der mindestens eine pharmazeutisch aktiven Wirkstoff (S)-Ketamin oder ein pharmazeutisch akzeptables Salz davon umfasst.

Der erfindungsgemäße orale Dünnfilm ist in einer anderen Ausführungsform bevorzugt dadurch gekennzeichnet, dass der mindestens eine pharmazeutisch aktiven Wirkstoff (R)-Ketamin oder ein pharmazeutisch akzeptables Salz davon umfasst.

Bevorzugt liegt Ketamin als HCl Salz oder als freie Base vor.

Unter Ketamin wird hierbei (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Ketamin wird nach Verabreichung zu Norketamin, Hydroxynorketamin und weiteren Substanzen metabolisiert.

Es kann sowohl (S)-Ketamin, als auch (R)-Ketamin, sowie eine racemische Mischung aus diesen beiden in der Matrixschicht des erfindungsgemäßen oralen Dünnfilms enthalten sein. Besonders bevorzugt liegt jedoch (S)-Ketamin als freie Base oder ein pharmazeutisch akzeptables Salz davon, insbesondere (S)-Ketamin·HCI, als einziges Stereoisomer von Ketamin vor, da die analgetische und anästhetische Potenz von (S)-Ketamin etwa dreifach höher als die der (R)-Form ist.

Der erfindungsgemäße orale Dünnfilm ist ferner bevorzugt dadurch gekennzeichnet, dass der mindestens eine pharmazeutisch aktive Wirkstoff in einer Menge von 45 bis 70 Gew.-%, vorzugsweise von 50 bis 65 Gew.-% oder von 55 bis 65 Gew.-% oder von 55 bis 60 Gew.-% oder von 60 bis 65 Gew.-%, bezogen auf das Gesamtgewicht der Matrixschicht, in der Matrixschicht vorhanden ist.

Vorzugsweise ist der erfindungsgemäße orale Dünnfilm ferner bevorzugt dadurch gekennzeichnet, dass der mindestens eine pharmazeutisch aktive Wirkstoff in einer Menge von 60 Gew.-%, bezogen auf das Gesamtgewicht der Matrixschicht, in der Matrixschicht vorhanden ist.

Der erfindungsgemäße orale Dünnfilm ist ferner bevorzugt dadurch gekennzeichnet, dass der mindestens eine pharmazeutisch aktive Wirkstoff in Form von Mikrokristallen vorliegt.

Geeignete mittlere Kristallgrößen dieser Mikrokristalle liegen vorzugsweise im Bereich von 1 bis 1000 µm oder im Bereich von 5 bis 500 µm oder im Bereich von 10 bis 200 µm. Besonders bevorzugt liegt die mittlere Kristallgröße im Bereich von 15 bis 25 µm, insbesondere im Bereich von 20 bis 22 µm. Die Kristallgröße kann beispielsweise mittels Lichtmikroskop oder mittels Röntgen-Mikro-Computertomographie (Mikro-CT) bestimmt werden.

Sofern nicht anders angegeben, beziehen sich alle genannten Molekulargewichte von Polymeren auf das gewichtsgemittelte Molekulargewicht (Mw), das mittels Gelpermeationschromatographie bestimmt wird.

Der erfindungsgemäße orale Dünnfilm ist ferner bevorzugt dadurch gekennzeichnet, dass der mindestens eine Polyvinylalkohol einen Polyvinylalkohol mit einem mittleren Molekulargewicht von etwa 25.000 bis etwa 250.000 g/mol umfasst.

Der erfindungsgemäße orale Dünnfilm ist ferner bevorzugt dadurch gekennzeichnet, dass der mindestens eine Polyvinylalkohol einen Polyvinylalkohol mit einem mittleren Molekulargewicht von etwa 25.000 bis etwa 35.000 g/mol und/oder einen Polyvinylalkohol mit einem mittleren Molekulargewicht von etwa 200.000 bis 210.000 g/mol umfasst.

Gemäß der vorliegenden Erfindung sind Polyvinylalkohole mit einem mittleren Molekulargewicht von etwa 31.000 (4-88) bis etwa 205.000 (40-88) g/mol besonders geeignet.

Gemäß der vorliegenden Erfindung sind Polyvinylalkohole mit einem mittleren Molekulargewicht mit etwa 31.000 (4-88) oder etwa 205.000 (40-88) g/mol besonders geeignet.

Ferner sind gemäß der vorliegenden Erfindung Polyvinylalkohole mit einer Viskosität von 3,4 bis 4,6 mPas (4-88) bis 34 bis 46 mPas (40-88) mPas in einer 40g/l wässrigen Lösung, bestimmt durch "falling ball method" (Ph.Eur. 2.2.49), besonders geeignet, oder Mischungen aus zwei oder mehr verschiedenen dieser PVA-Typen.

Ferner sind gemäß der vorliegenden Erfindung Polyvinylalkohole mit einer Viskosität von 3,4 bis 4,6 mPas (4-88) oder von 34 bis 46 mPas (40-88) mPas in einer 40g/l wässrigen Lösung, bestimmt durch "falling ball method" (Ph.Eur. 2.2.49), besonders geeignet, oder Mischungen aus zwei oder mehr verschiedenen dieser PVA-Typen.

Der erfindungsgemäße orale Dünnfilm ist ferner bevorzugt dadurch gekennzeichnet, dass das mindestens eine Polyvinylalkohol-Polyethylenglycol-Pfropfcopolymer, eine Polyethylenglycol-Hauptkette aufweist, auf die Polyvinylalkohol-Einheiten aufgepfropft sind.

Der erfindungsgemäße orale Dünnfilm ist ferner bevorzugt dadurch gekennzeichnet, dass das mindestens eine Polyvinylalkohol-Polyethylenglycol-Pfropfcopolymer, eine Polyethylenglycol-Hauptkette aufweist, auf die Polyvinylalkohol-Einheiten aufgepfropft sind, wobei das molare Verhältnis von Polyethylenglycol zu Polyvinylalkohol 1:3 beträgt.

Der erfindungsgemäße orale Dünnfilm ist ferner bevorzugt dadurch gekennzeichnet, dass das mindestens eine Polyvinylalkohol-Polyethylenglycol-Pfropfcopolymer, eine Polyethylenglycol-Hauptkette aufweist, auf die Polyvinylalkohol-Einheiten aufgepfropft sind, wobei das Polyvinylalkohol-Polyethylenglycol-Pfropfcopolymer ein mittleres Molekulargewicht im Bereich von 40.000 bis 50.000 g/mol, bevorzugt von etwa 45000 g/mol, aufweist.

Ein geeignetes und bevorzugtes Polyvinylalkohol-Polyethylenglycol-Pfropfcopolymer ist unter dem Handelsnamen Kollicoat IR (BASF) bekannt.

Der erfindungsgemäße orale Dünnfilm ist ferner bevorzugt dadurch gekennzeichnet, dass der mindestens eine Polyvinylalkohol in einer Menge von 5 bis 40 Gew.-%, vorzugsweise von 5 bis 20 Gew.-%, von 5 bis 19 Gew.-%, von 5 bis 18 Gew.-%, von 5 bis 17 Gew.-%, von 5 bis 16 Gew.-%, von 5 bis 15 Gew.-%, von 5 bis 14 Gew.-%, von 5 bis 13 Gew.-%, von 5 bis 12 Gew.-%, von 5 bis 11 Gew.-% oder von 5 bis 10 Gew.-%, bezogen auf das Gesamtgewicht der Matrixschicht, in der Matrixschicht vorhanden ist.

Der erfindungsgemäße orale Dünnfilm ist ferner bevorzugt dadurch gekennzeichnet, dass das mindestens eine Polyvinylalkohol-Polyethylenglycol-Pfropfcopolymer in einer Menge von 15 bis 45 Gew.-%, vorzugsweise von 17 bis 40 Gew.-% oder 20 bis 30 Gew.-%, bezogen auf das Gesamtgewicht der Matrixschicht, in der Matrixschicht vorhanden ist.

In einer anderen Ausführungsform ist der erfindungsgemäße orale Dünnfilm ferner bevorzugt dadurch gekennzeichnet, dass das mindestens eine Polyvinylalkohol-Polyethylenglycol-Pfropfcopolymer in einer Menge von 10 bis 30 Gew.-%, vorzugsweise von 15 bis 25 Gew.-%, von 17,5 bis 22,5 Gew.-% oder von 19 bis 21 Gew.-%, insbesondere etwa von 19,5 bis 20,5 Gew.-%, und besonders bevorzugt von etwa 20 Gew.-% oder von 20,1 Gew.-%, bezogen auf das Gesamtgewicht der Matrixschicht, in der Matrixschicht vorhanden ist.

Der erfindungsgemäße orale Dünnfilm ist ferner bevorzugt dadurch gekennzeichnet, dass die Matrixschicht ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge 0,1 bis 15 Gew.-%, bevorzugt von 0,1 bis 10 Gew.-% oder von 0,1 bis 5 Gew.-%, bezogen auf das Gesamtgewicht der Matrixschicht, in der Matrixschicht Schicht enthalten.

Vorzugsweise sind Süßstoffe, wie Saccharin Na und/oder Sucralose, in einer Gesamtmenge von 2 bis 5 Gew.-%, insbesondere etwa 3 Gew.-%, bezogen auf das Gesamtgewicht der Matrixschicht, in der Matrixschicht Schicht enthalten.

Vorzugsweise sind Aromastoffe, außer Süßstoffe, in einer Gesamtmenge von 2 bis 5 Gew.-%, insbesondere von etwa 3 Gew.-%, bezogen auf das Gesamtgewicht der Matrixschicht, in der Matrixschicht Schicht enthalten.

Vorzugsweise sind Farbstoffe in einer Gesamtmenge von 0,1 bis 1 Gew.-%, insbesondere von etwa 0,4 Gew.-%, bezogen auf das Gesamtgewicht der Matrixschicht, in der Matrixschicht Schicht enthalten.

Der erfindungsgemäße oralen Dünnfilm ist bezüglich seines Aufbaus keinen Beschränkungen unterworfen.

So kann der erfindungsgemäße oralen Dünnfilm als einschichtiger oraler Dünnfilm vorliegen und damit lediglich aus der Matrixschicht, wie vorstehend definiert, bestehen.

In einer anderen Ausführungsform kann der erfindungsgemäße oralen Dünnfilm als mehrschichtiger oraler Dünnfilm vorliegen und damit neben der Matrixschicht, wie vorstehend definiert, weitere Schichten enthalten.

Diese mehreren Schichten können direkt aufeinander laminiert werden oder mit einer dazwischenliegenden Klebeschicht verbunden werden.

Unter Klebeschicht wird eine Schicht verstanden, die als Klebstoff, wie in der DIN EN 923:2016-03 definiert, wirken kann. Eine nicht-klebende Schicht kann demnach nicht als Klebstoff wie vorstehend definiert wirken.

Als Klebeschichten eignen sich insbesondere wasserlösliche Klebeschichten, wie diese in der DE 10 2014 127 452 A1 beschrieben werden, deren diesbezüglicher Inhalt hiermit vollständig einbezogen wird.

Als weitere Schichten können beispielsweise Pufferschichten zur Einstellung eines pH-Werts oder langsam oder unlösliche Schichten vorliegen, die den oralen Dünnfilm vor vorzeitiger Erosion schützen.

Alternativ können weitere Matrixschichten vorliegen, die andere pharmazeutisch aktive Wirkstoffe oder Geschmacksstoffe bzw. geschmacksmaskierende Stoffe enthalten.

In einer Ausführungsform ist der erfindungsgemäße orale Dünnfilm dadurch gekennzeichnet, dass die Matrixschicht in Form eines glatten Films. Da bedeutet, dass die Matrixschicht beispielsweise nicht in Form eines Schaumes vorliegt.

Ein glatter Film ist vorzugsweise dadurch gekennzeichnet, dass ein glatter Film einen Volumenanteil von 0 bis 5 %, bezogen auf das Gesamtvolumen der Matrixschicht, an Blasen oder Hohlräumen aufweist. Die Hohlräume sind dabei vorzugsweise mit Luft oder einem Gas, vorzugsweise mit einem inerten Gas, besonders bevorzugt mit Stickstoff, Kohlendioxid, Helium oder einem Gemisch mindestens zweier dieser Gase, gefüllt. Der Durchmesser der Blasen oder Hohlräume liegt in der Regel im Bereich von 0,01 bis 350 µm. Besonders bevorzugt liegt der Durchmesser der Blasen oder Hohlräume im Bereich von 10 und 200 µm.

In einer anderen Ausführungsform ist der erfindungsgemäße orale Dünnfilm dadurch gekennzeichnet, dass die Matrixschicht in Form eines Hohlräume aufweisenden verfestigten Schaums vorliegt.

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung der Matrixschicht die transmukosale Resorption verbessert werden.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße mehrschichtige orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Matrixschicht. Auf diese Weise wird der vorteilhafte Effekt, die Lösung der Matrixschicht zu beschleunigen, in günstiger Weise beeinflusst.

Ferner können Matrixschicht zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Darreichungsform beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 350 µm liegen. Besonders bevorzugt liegt der Durchmesser im Bereich von 10 und 200 µm.

Der erfindungsgemäße orale Dünnfilm besitzt vorzugsweise eine Fläche von 0,5 cm² bis 10 cm², besonders bevorzugt von 2 cm² bis 8 cm² oder von 4 cm² bis 5 cm².

Das Flächengewicht der Matrixschicht, bzw. einer möglicherweise vorhandenen weiteren Schicht beträgt jeweils vorzugsweise mindestens 10 g/m², bevorzugter mindestens 20 g/m² oder mindestens 30 g/m² oder am meisten bevorzugt mindestens 50 g/m² oder von weniger als oder gleich 400 g/m², bevorzugter weniger als oder gleich 350 g/m² oder weniger als oder gleich 300 g/m² oder am meisten bevorzugt weniger als 250 g/m². Vorzugsweise beträgt das Flächengewicht 10 bis 400 g/m², bevorzugter 20 bis 350 g/m² oder 30 bis 300 g/m² und am meisten bevorzugt 50 bis 250 g/m².

Bevorzugt weist jede der vorhandenen Schichten, insbesondere die Matrixschicht, jeweils eine Schichtdicke von vorzugsweise 10 µm bis 500 µm, besonders bevorzugt von 20 µm bis 300 µm, auf.

Wenn die jeweiligen Schichten, insbesondere die Matrixschicht, in Form eines verfestigten Schaums vorliegen, so ist es bevorzugt, dass jede der als Schaum vorliegenden Schichten jeweils eine Schichtdicke von vorzugsweise 10 µm bis 3000 µm, besonders bevorzugt von 90 µm bis 2000 µm, aufweist.

Der erfindungsgemäße orale Dünnfilm ist ferner bevorzugt dadurch gekennzeichnet, dass der mindestens eine pharmazeutisch aktiven Wirkstoff Ketamin, vorzugsweise als freie Base oder als Ketamin HCl, in einer Gesamtmenge von 25 mg bis 150 mg, vorzugsweise von 25 mg bis 125 mg, insbesondere von etwa 50 mg bis 150 mg, umfasst.

Insbesondere ist der erfindungsgemäße orale Dünnfilm dadurch gekennzeichnet, dass der mindestens eine pharmazeutisch aktiven Wirkstoff Ketamin, vorzugsweise als freie Base oder als Ketamin HCl, in einer Gesamtmenge von 50 mg bis 100 mg, vorzugsweise von etwa 50 mg oder etwa 100 mg, in der Matrixschicht vorliegt.

Der erfindungsgemäße orale Dünnfilm ist ferner bevorzugt dadurch gekennzeichnet, dass der mindestens eine pharmazeutisch aktiven Wirkstoff als Ketamin, vorzugsweise als freie Base oder als Ketamin HCl, in einer Gesamtmenge von 25 mg bis 150 mg, vorzugsweise von 25 mg bis 125 mg, insbesondere von etwa 50 mg bis 150 mg, in der Matrixschicht vorliegt.

Insbesondere ist der erfindungsgemäße orale Dünnfilm dadurch gekennzeichnet, dass der mindestens eine pharmazeutisch aktive Wirkstoff als Ketamin, vorzugsweise als freie Base oder als Ketamin HCl, in einer Gesamtmenge von 50 mg bis 100 mg, vorzugsweise von etwa 50 mg oder etwa 100 mg, in der Matrixschicht vorliegt.

Der erfindungsgemäße orale Dünnfilm ist ferner bevorzugt dadurch gekennzeichnet, dass der mindestens eine pharmazeutisch aktiven Wirkstoff als Ketamin, vorzugsweise als freie Base oder als Ketamin HCl, in einer Gesamtmenge von 2 mg oder 5 mg oder 7 mg oder 10 mg oder 15 mg oder 20 mg oder 25 mg oder 30 mg oder 35 mg oder 40 mg oder 45 mg oder 50 mg oder 55 mg oder 60 mg oder 65 mg oder 70 mg oder 80 mg oder 90 mg oder 95 mg oder 100 mg oder 105 mg oder 110 mg oder 115 mg oder 120 mg oder 125 mg oder 130 mg oder 135 mg oder 140 mg oder 145 mg oder 150 mg in der Matrixschicht vorliegt.

Der erfindungsgemäße orale Dünnfilm ist ferner bevorzugt dadurch gekennzeichnet, dass die Durchstoßfestigkeit mindestens 0,15 N/mm², vorzugsweise mindestens 0,18 N/mm², besonders bevorzugt 0,20 N/mm² oder mehr, beträgt. Das Flächengewicht beträgt hierbei vorzugsweise 150 bis 250 g/m², besonders bevorzugt 180 bis 220 g/m²

Die Durchstoßfestigkeit wird vorzugsweise wie folgt bestimmt:
Verwendetes Prüfgerät: Kraftmessgerät Sauter FH-20.
Prüffläche: runde Prüffläche mit Durchmesser 5 mm.

### Durchführung:

Das Kraftmessgerät wird fixiert und ein 10 cm² Laminatprüfling wird mittig an die Prüffläche des Gerätes (runde Prüffläche mit Durchmesser 5 mm) angelegt. Der Laminatprüfling wird an den Rändern fixiert und eine Kraft Richtung Prüfkörper ausgeübt, die Kraft wird gesteigert bis der der Laminatprüfling durchstoßen wurde. Es wird der resultierende Maximalwert der Kraft gemessen, der auf den Prüfkörper bis zum Durchstoßen eingewirkt hat. Die Messung wird mit n=3 Laminatprüflingen pro Laminatcharge durchgeführt.

Der erfindungsgemäße orale Dünnfilm ist ferner bevorzugt dadurch gekennzeichnet, dass die Bioverfügbarkeit des mindestens einen pharmazeutisch aktiven Wirkstoffs, insbesondere des Ketamins, vorzugsweise als freie Base oder als Ketamin HCl, mindestens 5 % oder mindestens 10 % oder mindestens 15 % oder mindestens 20% oder mindestens 25 % oder mindestens 30 % oder mindestens 35 % oder mindestens 40% oder mindestens 45 % oder mindestens 50% oder mindestens 55% oder mindestens 60 % oder mindestens 65 % oder mindestens 70% oder mindestens 75% oder mindestens 80 % oder mindestens 85 % oder mindestens 90 % oder mindestens 95 % oder mindestens 97 % oder mindestens 99 % beträgt.

Der erfindungsgemäße orale Dünnfilm ist ferner vorzugsweise durch die folgenden Freisetzungsraten gekennzeichnet, wobei sich die Freisetzungsrate auf die Freisetzung des mindestens einen pharmazeutisch aktiven Wirkstoffs nach einer gewissen Zeit nach Applikation des erfindungsgemäßen oralen Dünnfilms bezieht.

Es ist bevorzugt, wenn nach 1 min mindestens 40 % oder mindestens 50 % des mindestens einen pharmazeutisch aktiven Wirkstoffs freigesetzt sind.

Es ist bevorzugt, wenn nach 2 min mindestens 75 % oder mindestens 80 % oder mindestens 85 % des mindestens einen pharmazeutisch aktiven Wirkstoffs freigesetzt sind.

Es ist bevorzugt, wenn nach 15 s etwa 5 bis 10 % des mindestens einen pharmazeutisch aktiven Wirkstoffs freigesetzt sind.

Es ist bevorzugt, wenn nach 30 s etwa 20 bis 25 % des mindestens einen pharmazeutisch aktiven Wirkstoffs freigesetzt sind.

Es ist bevorzugt, wenn nach 45 s etwa 30 bis 40 % des mindestens einen pharmazeutisch aktiven Wirkstoffs freigesetzt sind.

Es ist bevorzugt, wenn nach 1 min etwa 50 bis 60 % des mindestens einen pharmazeutisch aktiven Wirkstoffs freigesetzt sind.

Es ist bevorzugt, wenn nach 1 min und 15 s etwa 60 bis 70 % des mindestens einen pharmazeutisch aktiven Wirkstoffs freigesetzt sind.

Es ist bevorzugt, wenn nach 1 min und 30 s etwa 70 bis 80 % des mindestens einen pharmazeutisch aktiven Wirkstoffs freigesetzt sind.

Es ist bevorzugt, wenn nach 1 min und 45 s etwa 80 bis 85 % des mindestens einen pharmazeutisch aktiven Wirkstoffs freigesetzt sind.

Es ist bevorzugt, wenn nach 2 min etwa 82 bis 88 % des mindestens einen pharmazeutisch aktiven Wirkstoffs freigesetzt sind.

Es ist bevorzugt, wenn nach 2 min und 15 s etwa 84 bis 90 % des mindestens einen pharmazeutisch aktiven Wirkstoffs freigesetzt sind.

Es ist bevorzugt, wenn nach 2 min und 30 s etwa 86 bis 92 % des mindestens einen pharmazeutisch aktiven Wirkstoffs freigesetzt sind.

Im Folgenden werden bevorzugte Ausführungsformen hinsichtlich der maximalen Plasmakonzentration (Cmax) des Wirkstoffs bzw. Metaboliten davon für den erfindungsgemäßen oralen Dünnfilm beschrieben, bei dem (S)-Ketamin als pharmazeutisch aktiver Wirkstoff eingesetzt wird.

100 mg (S)-Ketamin können dabei in einer Dosis oder mittels zwei Dosen von jeweils 50 mg (S)-Ketamin verabreicht werden.

Der erfindungsgemäße oraler Dünnfilm ist vorzugsweise dadurch gekennzeichnet, dass die maximale Plasmakonzentration an (S)-Ketamin nach Verabreichung einer Dosis von 50 mg (S)-Ketamin bei 50 bis 200 ng/mL liegt.

Der erfindungsgemäße oraler Dünnfilm ist ferner vorzugsweise dadurch gekennzeichnet, dass die maximale Plasmakonzentration des Ketaminmetaboliten (S)-Norketamin nach Verabreichung einer Dosis von 50 mg (S)-Ketamin bei 200 bis 400 ng/mL liegt.

Der erfindungsgemäße oraler Dünnfilm ist ferner vorzugsweise dadurch gekennzeichnet, dass die maximale Plasmakonzentration des Ketaminmetaboliten (S)-Hydroxynorketamin nach Verabreichung einer Dosis von 50 mg (S)-Ketamin bei 50 bis 150 ng/mL liegt.

Der erfindungsgemäße oraler Dünnfilm ist vorzugsweise dadurch gekennzeichnet, dass die maximale Plasmakonzentration an (S)-Ketamin nach Verabreichung einer Dosis von 100 mg (S)-Ketamin bei 100 bis 200 ng/mL liegt.

Der erfindungsgemäße oraler Dünnfilm ist ferner vorzugsweise dadurch gekennzeichnet, dass die maximale Plasmakonzentration des Ketaminmetaboliten (S)-Norketamin nach Verabreichung einer Dosis von 100 mg (S)-Ketamin bei 300 bis 500 ng/mL liegt.

Der erfindungsgemäße oraler Dünnfilm ist ferner vorzugsweise dadurch gekennzeichnet, dass die maximale Plasmakonzentration des Ketaminmetaboliten (S)-Hydroxynorketamin nach Verabreichung einer Dosis von 100 mg (S)-Ketamin bei 100 bis 250 ng/mL liegt.

Der erfindungsgemäße oraler Dünnfilm ist vorzugsweise dadurch gekennzeichnet, dass die maximale Plasmakonzentration an (S)-Ketamin nach Verabreichung einer Dosis von 50 mg (S)-Ketamin bei 70 bis 120 ng/mL liegt.

Der erfindungsgemäße oraler Dünnfilm ist ferner vorzugsweise dadurch gekennzeichnet, dass die maximale Plasmakonzentration des Ketaminmetaboliten (S)-Norketamin nach Verabreichung einer Dosis von 50 mg (S)-Ketamin bei 200 bis 300 ng/mL liegt.

Der erfindungsgemäße oraler Dünnfilm ist ferner vorzugsweise dadurch gekennzeichnet, dass die maximale Plasmakonzentration des Ketaminmetaboliten (S)-Hydroxynorketamin nach Verabreichung einer Dosis von 50 mg (S)-Ketamin bei 70 bis 120 ng/mL liegt.

Der erfindungsgemäße oraler Dünnfilm ist vorzugsweise dadurch gekennzeichnet, dass die maximale Plasmakonzentration an (S)-Ketamin nach Verabreichung einer Dosis von 100 mg (S)-Ketamin bei 120 bis 160 ng/mL liegt.

Der erfindungsgemäße oraler Dünnfilm ist vorzugsweise dadurch gekennzeichnet, dass die maximale Plasmakonzentration des Ketaminmetaboliten (S)-Norketamin nach Verabreichung einer Dosis von 100 mg (S)-Ketamin bei 300 bis 350 ng/mL liegt.

Der erfindungsgemäße oraler Dünnfilm ist vorzugsweise dadurch gekennzeichnet, dass die maximale Plasmakonzentration des Ketaminmetaboliten (S)-Hydroxynorketamin nach Verabreichung einer Dosis von 100 mg (S)-Ketamin bei 150 bis 220 ng/mL liegt.

Der erfindungsgemäße oraler Dünnfilm ist vorzugsweise dadurch gekennzeichnet, dass die maximale Plasmakonzentration an (S)-Ketamin nach sublingualer Verabreichung einer Dosis von 50 mg (S)-Ketamin bei 70 bis 120 ng/mL liegt.

Der erfindungsgemäße oraler Dünnfilm ist vorzugsweise dadurch gekennzeichnet, dass die maximale Plasmakonzentration des Ketaminmetaboliten (S)-Norketamin nach sublingualer Verabreichung einer Dosis von 50 mg (S)-Ketamin bei 200 bis 300 ng/mL liegt.

Der erfindungsgemäße oraler Dünnfilm ist vorzugsweise dadurch gekennzeichnet, dass die maximale Plasmakonzentration des Ketaminmetaboliten (S)-Hydroxynorketamin nach sublingualer Verabreichung einer Dosis von 50 mg (S)-Ketamin bei 70 bis 120 ng/mL liegt.

Der erfindungsgemäße oraler Dünnfilm ist vorzugsweise dadurch gekennzeichnet, dass die maximale Plasmakonzentration an (S)-Ketamin nach sublingualer Verabreichung einer Dosis von 100 mg (S)-Ketamin bei 120 bis 160 ng/mL liegt.

Der erfindungsgemäße oraler Dünnfilm ist vorzugsweise dadurch gekennzeichnet, dass die maximale Plasmakonzentration des Ketaminmetaboliten (S)-Norketamin nach sublingualer Verabreichung einer Dosis von 100 mg (S)-Ketamin bei 300 bis 350 ng/mL liegt.

Der erfindungsgemäße oraler Dünnfilm ist vorzugsweise dadurch gekennzeichnet, dass die maximale Plasmakonzentration des Ketaminmetaboliten (S)-Hydroxynorketamin nach sublingualer Verabreichung einer Dosis von 100 mg (S)-Ketamin bei 150 bis 220 ng/mL liegt.

Der erfindungsgemäße oraler Dünnfilm ist vorzugsweise dadurch gekennzeichnet, dass die maximale Plasmakonzentration an (S)-Ketamin nach bukkaler Verabreichung einer Dosis von 50 mg (S)-Ketamin bei 80 bis 160 ng/mL liegt.

Der erfindungsgemäße oraler Dünnfilm ist vorzugsweise dadurch gekennzeichnet, dass die maximale Plasmakonzentration des Ketaminmetaboliten (S)-Norketamin nach bukkaler Verabreichung einer Dosis von 50 mg (S)-Ketamin bei 200 bis 280 ng/mL liegt.

Der erfindungsgemäße oraler Dünnfilm ist vorzugsweise dadurch gekennzeichnet, dass die maximale Plasmakonzentration des Ketaminmetaboliten (S)-Hydroxynorketamin nach bukkaler Verabreichung einer Dosis von 50 mg (S)-Ketamin bei 60 bis 100 ng/mL liegt.

Der erfindungsgemäße oraler Dünnfilm ist vorzugsweise dadurch gekennzeichnet, dass die maximale Plasmakonzentration an (S)-Ketamin nach bukkaler Verabreichung einer Dosis von 100 mg (S)-Ketamin bei 120 bis 200 ng/mL liegt.

Der erfindungsgemäße oraler Dünnfilm ist vorzugsweise dadurch gekennzeichnet, dass die maximale Plasmakonzentration des Ketaminmetaboliten (S)-Norketamin nach bukkaler Verabreichung einer Dosis von 100 mg (S)-Ketamin bei 400 bis 500 ng/mL liegt.

Der erfindungsgemäße oraler Dünnfilm ist vorzugsweise dadurch gekennzeichnet, dass die maximale Plasmakonzentration des Ketaminmetaboliten (S)-Hydroxynorketamin nach bukkaler Verabreichung einer Dosis von 50 mg (S)-Ketamin bei 120 bis 200 ng/mL liegt.

Der erfindungsgemäße orale Dünnfilm ist ferner bevorzugt dadurch gekennzeichnet, dass die Matrixschicht 60 Gew.-% (S)-Ketamin HCl, 10 Gew.- des Polyvinylalkohols 40-88, wie vorstehend definiert und 20 Gew.-% bzw. 20,1 Gew.-% eines Polyvinylalkohol-Polyethylenglycol-Pfropfcopolymers, bevorzugt wie vorstehend definiert, umfasst.

Der erfindungsgemäße orale Dünnfilm ist ferner bevorzugt dadurch gekennzeichnet, dass die Matrixschicht 60 Gew.-% (S)-Ketamin HCl, 10 Gew.- des Polyvinylalkohols 40-88, wie vorstehend definiert, und 20,1 Gew.-% eines Polyvinylalkohol-Polyethylenglycol-Pfropfcopolymers, bevorzugt wie vorstehend definiert, 1,0 Gew.-% Saccharin Na, 2,0 Gew.-% Sucralose, 3,5 Gew.-% Glycerol, 3,0 Gew.-% eines pharmazeutisch akzeptablen Geschmacksstoffes und 0,4 Gew.-% eines pharmazeutisch akzeptablen Farbstoffes umfasst. Als Lösungsmittel dient dabei vorzugsweise reines Wasser.

In einer ganz besonders bevorzugten Ausführungsform weist der orale Dünnfilm eine Formulierung gemäß Formulierung 15 der Tabelle 3 auf.

Der erfindungsgemäße orale Dünnfilm kann mit üblichen Verfahren hergestellt werden.

Die vorstehenden Definitionen bezüglich des oralen Dünnfilms gelten analog für das erfindungsgemäße Verfahren.

Vorzugsweise umfasst ein Verfahren zur Herstellung des erfindungsgemäßen oralen Dünnfilms die Schritte:
a) Herstellen einer Lösung, Dispersion oder Schmelze, umfassend den mindestens einen pharmazeutisch aktiven Wirkstoff, den mindestens einen Polyvinylalkohol und das mindestens eine Polyvinylalkohol-Polyethylenglycol-Pfropfcopolymer;
a1) optional Aufschäumen der Lösung, Dispersion oder Schmelze aus Schritt a) durch Eintragen eines Gases oder Gasgemisches, durch chemische Gaserzeugung oder durch Entspannen eines gelösten Gases,
b) Ausstreichen der Lösung, Dispersion oder Schmelze aus Schritt a) bzw. der optional aufgeschäumten Lösung, Dispersion oder Schmelze aus Schritt a1.

Dem Fachmann ist klar, dass der Schritt a1) nur dann notwendig ist, wenn die Matrixschicht in Form eines Hohlräume aufweisenden verfestigten Schaums vorliegen soll.

Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann.

Die vorliegende Erfindung betrifft ferner einen oralen Dünnfilm erhältlich nach dem vorstehend beschriebenen Verfahren.

Außerdem betrifft die vorliegende Erfindung einen oralen Dünnfilm, wie vorstehend beschrieben, oder erhältlich nach dem vorstehend beschriebenen Verfahren zur Verwendung als Arzneimittel.

Zudem betrifft die vorliegende Erfindung einen oralen Dünnfilm, wie vorstehend beschrieben, oder erhältlich nach dem vorstehend beschriebenen Verfahren zur Verwendung als Arzneimittel zur sublingualen und/oder bukkalen Verabreichung.

Außerdem betrifft die vorliegende Erfindung einen oralen Dünnfilm, wie vorstehend beschrieben, oder erhältlich nach dem vorstehend beschriebenen Verfahren zur Verwendung in der Behandlung von Schmerzen und/oder Depressionen.

Außerdem betrifft die vorliegende Erfindung einen oralen Dünnfilm, wie vorstehend beschrieben, oder erhältlich nach dem vorstehend beschriebenen Verfahren zur Verwendung in der Behandlung von Schmerzen und/oder Depressionen durch sublinguale und/oder bukkale Verabreichung des oralen Dünnfilms.

Die vorliegende Erfindung betrifft zudem einen oralen Dünnfilm, wie vorstehend beschrieben oder erhältlich nach dem vorstehend beschriebenen Verfahren, wobei Ketamin, vorzugsweise (S)-Ketamin, bzw. ein pharmazeutisch akzeptables Salz davon, als pharmazeutisch aktiver Wirkstoff in der Matrixschicht eingesetzt wird, zur Verwendung in der Behandlung von Schmerzen und/oder Depressionen, insbesondere zur Reduzierung des Suizidrisikos, und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorliegende Erfindung betrifft insbesondere einen oralen Dünnfilm, wie vorstehend beschrieben oder erhältlich nach dem vorstehend beschriebenen Verfahren, wobei Ketamin, vorzugsweise (S)-Ketamin, bzw. ein pharmazeutisch akzeptables Salz davon, als pharmazeutisch aktiver Wirkstoff in der Matrixschicht eingesetzt wird, zur Verwendung in der Behandlung von Schmerzen, vorzugsweise wie nachstehend definiert.

Als Schmerz wird in der Regel ein schmerzhaftes Gefühl bezeichnet, das oft durch intensive oder schädliche Reize verursacht wird. Schmerzen, die lange andauern, werden als chronisch oder anhaltend bezeichnet und Schmerzen, die schnell verschwinden, werden als akut bezeichnet.

Nozizeptive Schmerzen werden durch die Stimulation von sensorischen Nervenfasern verursacht, die auf Reize reagieren, die sich einer schädlichen Intensität nähern oder diese überschreiten (Nozizeptoren), und können nach dem Modus der schädlichen Stimulation klassifiziert werden. Die gebräuchlichsten Kategorien sind thermische, mechanische und chemische Stimulation. Einige Nozizeptoren reagieren auf mehr als eine dieser Modalitäten und werden daher als polymodal bezeichnet.

Nozizeptive Schmerzen können auch in "viszerale" "tief somatische" und "oberflächliche somatische" Schmerzen unterteilt werden.

Neuropathischer Schmerz wird in der Regel durch eine Schädigung oder Erkrankung verursacht, die einen Teil des Nervensystems betrifft, der an den Körpergefühlen beteiligt ist (das somatosensorische System). Neuropathische Schmerzen können in periphere, zentrale oder gemischte (periphere und zentrale) neuropathische Schmerzen unterteilt werden. Periphere neuropathische Schmerzen werden oft als "brennend", "kribbelnd", "elektrisch" oder "stechend" beschrieben.

Die Erfindung wird nachfolgend anhand von nicht beschränkenden Beispielen näher erläutert.

### Beispiele:

### Beispiel 1:

In den folgenden Beispielen wurden die in Tabelle 1 genannten Ausgangsmaterialien eingesetzt.

**Tabelle 1:**

| **Ausgangsmaterial** | **Funktion** |
|---|---|
| (S)-Ketamin HCl | API |
| Polyvinylalkohol | Matrixpolymer |
| (PVA) 4-88 | |
| (35% Lösung) | |
| Polyvinylalkohol | Matrixpolymer |
| (PVA) 40-88 | |
| (15% Lösung) | |
| Kollicoat IR | Matrixpolymer |
| (30% Lösung) | |
| Glycerol | Weichmacher |
| Sucralose | Süßstoff |
| Saccharin Na | Süßstoff |
| Cherry Flavor | Geschmacksstoff |
| M55394 (EU taste) | |
| FD&C Red 40 | Farbstoff |
| Gereinigtes Wasser | Prozesslösungsmittel |
| P 120 g/m² wei PE2 AB1 | Beschichtungsliner |

Aus diesen Ausgangsmaterialien wurden gemäß dem erfindungsgemäßen Verfahren mehrere orale Dünnfilme hergestellt und untersucht. Die Zusammensetzung dieser ist in Tabelle 2 angegeben.

**Tabelle 2:**

| **Material** | **1 [Gew.-%]** | **2 [Gew.-%]** | **3 [Gew.-%]** | **4 [Gew.-%]** | **5 [Gew.-%]** | **6 [Gew.-%]** | **7 [Gew.-%]** | **8 [Gew.-%]** | **9 [Gew.-%]** | **10 [Gew.-%]** |
|---|---|---|---|---|---|---|---|---|---|---|
| (S)-Ketamin HCl | 55,0 | 50,0 | 50,0 | 60,0 | 60,0 | 55,0 | 60,0 | 65,0 | 60,0 | 60,0 |
| PVA 4-88 | 35,0 | -- | -- | 30,5 | -- | -- | -- | -- | -- | -- |
| PVA 40-88 | -- | -- | 9,9 | -- | 7,6 | 8,8 | 9,9 | 6,4 | -- | 2,0 |
| Kollicoat IR | -- | 39,5 | 29,6 | -- | 22,9 | 26,2 | 20,6 | 19,1 | 30,5 | 28,5 |
| Saccharin Na | 1,0 | 1,0 | 1,0 | 1,0 | 1,0 | 1,0 | 1,0 | 1,0 | 1,0 | 1,0 |
| Sucralose | 2,0 | 2,0 | 2,0 | 2,0 | 2,0 | 2,0 | 2,0 | 2,0 | 2,0 | 2,0 |
| Glycerol | 4,0 | 4,0 | 4,5 | 3,5 | 3,5 | 4,0 | 3,5 | 3,5 | 3,5 | 3,5 |
| Cherry flavor M55394 | 3,0 | 3,0 | 3,0 | 3,0 | 3,0 | 3,0 | 3,0 | 3,0 | 3,0 | 3,0 |
| **Lösungsmittel** | Aq. Pur. | Aq. Pur. | Aq. Pur. | Aq. Pur. | Aq. Pur. | Aq. Pur. | Aq. Pur. | Aq. Pur. | Aq. Pur. | Aq. Pur. |
| **Schaum/Film** | Schaum | Film | Schaum | Schaum | Schaum | Schaum | Schaum | Schaum | Film | Schaum |

Alle erfindungsgemäßen oralen Dünnfilme mit den in Tabelle 2 aufgeführten Zusammensetzungen lösen die der Erfindung zugrundeliegende Aufgabe.

Basierend auf der Zusammensetzung 2 wurden weitere orale Dünnfilme mit der Zusammensetzung gemäß Tabelle 3 hergestellt und geprüft.

**Tabelle 3:**

| **Material** | **11 [Gew.-%]** | **12 [Gew.-%]** | **13 [Gew.-%]** | **14 [Gew.-%]** | **15 [Gew.-%]** |
|---|---|---|---|---|---|
| (S)-Ketamin HCl | 60,0 | 55,0 | 60,0 | 60,0 | 60,0 |
| PVA 4-88 | -- | -- | 5,0 | 10,0 | -- |
| PVA 40-88 | 5,0 | 5,0 | -- | -- | 10,0 |
| Kollicoat IR | 25,1 | 30,1 | 25,1 | 20,1 | 20,1 |
| Saccharin Na | 1,0 | 1,0 | 1,0 | 1,0 | 1,0 |
| Sucralose | 2,0 | 2,0 | 2,0 | 2,0 | 2,0 |
| Glycerol | 3,5 | 3,5 | 3,5 | 3,5 | 3,5 |
| Cherry flavor M55394 | 3,0 | 3,0 | 3,0 | 3,0 | 3,0 |
| FD&C Red No, 40 | 0,4 | 0,4 | 0,4 | 0,4 | 0,4 |
| **Lösungsmittel** | Aq. Pur. | Aq. Pur. | Aq. Pur. | Aq. Pur. | Aq. Pur. |
| **Prüfung** | Guter Film, kleine Partikel sichtbar | Guter Film, Blasen sichtbar, raue Oberfläche | Guter Film, Blasen sichtbar, raue Oberfläche | Guter Film, kleine Partikel sichtbar | Sehr guter Film, wenige Blasen, sehr gute Reißfestigkeit |

Alle oralen Dünnfilm mit den in Tabelle 3 aufgeführten Zusammensetzungen lösen die der Erfindung zugrundeliegende Aufgabe.

Die Formulierung 15 wurde weitergehend untersucht.

Die Formulierung 15 ist 9 Monate bei 25°C/60 r.F. % - 40°/r.F. 75 % stabil.

Es wurde der Restwassergehalt der Formulierung 15 mittels Karl-Fischer Titration bestimmt.

Es wurde der Restwassergehalt von 6 Proben bestimmt. Der ermittelte Restwassergehalt betrug 4,40 Gew.-%, 3,82 Gew.-%, 4,02 Gew.-%, 4,51 Gew.-%, 4,91 Gew.-% und 4,57 Gew.-%.

Dieser Restwassergehalt ist akzeptabel.

Es wurde die Zerfallszeit der Formulierung 15 in einem 1L Becherglas mit 900 ml demineralisiertem Wasser (temperiert auf 37°C ± 2°C) bestimmt.

Es wurde die Zerfallszeit von sechs Proben bestimmt. Die ermittelte Zerfallszeit betrug 52 s, 55 s, 58 s, 56 s, 54 s und 55 s.

Die Formulierung 15 weist damit eine gute Zerfallszeit auf.

Ferner wurde die in vitro Freisetzung des Wirkstoffs aus der Formulierung 15 bestimmt.

Bei der in vitro Freisetzung wird (S)-Ketamin aus (S)-Ketamin HCl haltigen oralen Dünnfilmen freigesetzt und bestimmt. Der Wirkstoff wird in Phosphatpuffer pH 6.8 USP freigesetzt und dann durch in-Situ Fiber Optik UV-System bestimmt. Die Quantifizierung erfolgte gegen einen externen Standard.

Die Freisetzung wird mit Dissolution Apparatus 2 - (Paddle over sinker) gemäß USP <711> durchgeführt.

**Tabelle 4:**

| | |
|---|---|
| Sinker: | Stainless Steel Capsule Sinker with 10 Spirals, 31.0 x 11.0 mm Capacity (Sotax Style) |
| Rührgeschwindigkeit: | 50 rpm |
| Abstand zwischen dem Vesselboden und der Unterkante des Paddle: | 25 mm ± 2 mm |
| Temperatur: | 37°C ± 0,5°C |
| Freisetzungsmedium: | Phosphatpuffer pH 6,8 USP |
| Volumen Freisetzungsmedium: | 500 mL |
| Probenmesszeitpunkte: | Alle 5 Sekunden (0 bis 5 min) |
| | Alle 10 Sekunden (5 bis 10 min) |
| | Alle 15 Sekunden (10 bis 15 min) |

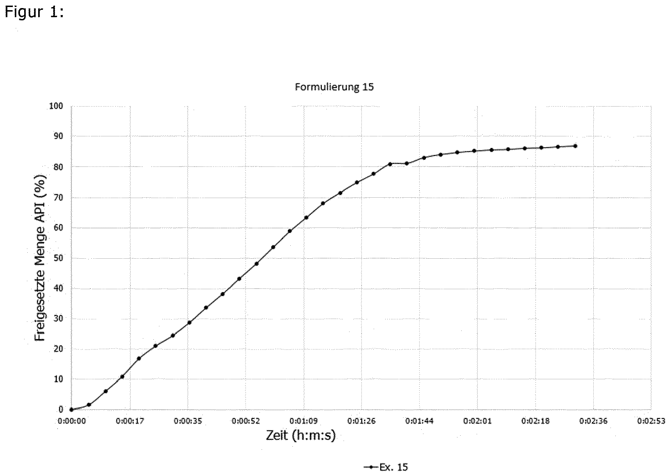

Die Ergebnisse der Freisetzungsstudie sind in Figur 1 dargestellt und betrugen:

| | |
|---|---|
| nach 15s | ca. 11% |
| nach 30s | ca. 24% |
| nach 45s | ca. 38% |
| nach 1 Minute | ca. 54% |
| nach 1 Minute 15s | ca. 68% |
| nach 1 Minute 30s | ca. 78% |
| nach 1 Minute 45s | ca. 83% |
| nach 2 Minuten | ca. 85% |
| nach 2 Minuten 15s | ca. 86% |
| nach 2 Minuten 30s | ca. 87% |

### Beispiel 2

Die Formulierungen aus Tabelle 3 wurde in weiteren Studien näher untersucht.

### 1. Messung der Durchstoßfestigkeit (puncture strength)

Durchführung der Messung (Prüfung Durchstoßfestigkeit Laminat):
Verwendetes Prüfgerät: Kraftmessgerät Sauter FH-20.
Prüffläche: runde Prüffläche mit Durchmesser 5 mm.

Als Vergleichsprodukt wurde das kommerziell erhältliche Produkt "LISTERINE POCKETPAKS^{®} COOL MINT ORAL CARE FRESH BREATH STRIPS" eingesetzt. Dieses enthält Pullulan, Menthol, Kalium-Acesulfam, Kupfergluconat, Polysorbat 80, Chondrus Crispus Gum (Carrageen), Glyceryloleat, Thymol, Eucalyptol, Methylalicylat, Ceratonia Siliqua Gum (Johannisbrotkernmehl), Propylenglycol, Xanthan Gum, Aroma (Flavor), FD&C Blue No. 1 (Farbstoff) und FD&C Green No. 3 (Farbstoff).

### Durchführung:

Das Kraftmessgerät wurde fixiert und ein 10 cm² Laminatprüfling (für Listerine wurde ein 32 mm x 22 mm Listerine OTF verwendet) wurde mittig an die Prüffläche des Gerätes (runde Prüffläche mit Durchmesser 5 mm) angelegt. Der Laminatprüfling wurde an den Rändern fixiert und eine Kraft Richtung Prüfkörper ausgeübt, die Kraft wurde gesteigert bis der Laminatprüfling durchstoßen wurde. Es wurde der resultierende Maximalwert der Kraft gemessen, der auf den Prüfkörper bis zum Durchstoßen eingewirkt hat. Die Messung wurde mit n=3 Laminatprüflingen pro Laminatcharge durchgeführt (für Listerine n=3 Messung mit OTF).

Die Ergebnisse sind in nachfolgender Tabelle 5 zusammengefasst:

**Tabelle 5:**

| **Formulierung** | **Durchschnitt** | **Standardabweichung** | **Flächengewicht** | **Durchstoßfestigkeit** |
|---|---|---|---|---|
| | [N] | [N] | [g/m²] | [N/mm²] |
| 2 | 1,96 | 0,09 | 150 | 0,10 |
| 3 | 2,22 | 0,10 | 130 | 0,11 |
| 5 | 1,85 | 0,06 | 157 | 0,09 |
| 5 | 2,88 | 0,19 | 206 | 0,15 |
| 7 | 2,92 | 0,08 | 167 | 0,15 |
| 8 | 1,27 | 0,12 | 145 | 0,06 |
| 9 | 1,55 | 0,12 | 190 | 0,08 |
| 10 | 1,79 | 0,15 | 156 | 0,09 |
| 11 | 2,68 | 0,20 | 188 | 0,14 |
| 12 | 2,63 | 0,05 | 172 | 0,13 |
| 13 | 2,04 | 0,05 | 184 | 0,10 |
| 14 | 2,26 | 0,11 | 180 | 0,12 |
| 15 | 4,00 | 0,15 | 206 | 0,20 |
| Listerine (Vergleich) | 3,14 | 0,80 | 46,3 | 0,16 |

Alle gezeigten Beispiele weisen trotz hoher Wirkstoffbeladung eine gute Durchstoßfestigkeit auf (vergleichbar mit dem Marktprodukt Listerine als Referenz).

### Beispiel 3:

Wie in der Beschreibung ausgeführt, ist der erfindungsgemäße orale Dünnfilm bevorzugt dadurch gekennzeichnet, dass der mindestens eine pharmazeutisch aktive Wirkstoff, vorzugsweise Ketamin, in Form von Mikrokristallen vorliegt. Geeignete mittlere Kristallgrößen dieser Mikrokristalle liegen vorzugsweise im Bereich von 1 bis 1000 µm oder im Bereich von 5 bis 500 µm oder im Bereich von 10 bis 200 µm. Die Kristallgröße kann beispielsweise mittels Lichtmikroskop oder mittels Röntgen-Mikro-Computertomographie (Mikro-CT) bestimmt werden.

Prüfmethode und Messbedingungen Röntgen-Mikro-Computertomographie (Mikro-CT):
Instrument SkyScan 2211
Röntgenenergie 60 kV
Auflösung 0.75 µm/voxel
Rekonstruktion nach Feldkamp

Die Ergebnisse sind in der nachfolgenden Tabelle 6 zusammengefasst:

**Tabelle 6:**

| **Formulierung** | **Kristallgröße und Standardabweichung** |
|---|---|
| 4 | 20,1 µm +- 7,2 µm |
| 7 | 18,4 µm +- 6,8 µm |
| 14 | 16,9 µm +- 6,2 µm |
| 15 | 21,0 µm +- 7,8 µm |

### Beispiel 4:

Ergebnisse einer klinischen Studie mit einem OTF basierend auf der Formulierung 15 gemäß Tabelle 3.

### Zielsetzung:

Primäres Ziel: Bestimmung des pharmakokinetischen Profils eines oralen Dünnfilms von (S)-Ketamin mit 50 mg (S)-Ketamin; Sekundärziel: (1) Bestimmung des pharmakodynamischen Profils eines oralen Dünnfilms von (S)-Ketamin mit 50 oder 100 mg (S)-Ketamin mit den Endpunkten Antinozizeption und psychomimetische Nebenwirkungen; (2) Bestimmung der Sicherheit und Verträglichkeit des oralen Dünnfilms von (S)-Ketamin.

### Studiendesign:

Die Studie hatte ein exploratives, offenes, crossover und randomisiertes Design. Alle Probanden wurden zweimal behandelt. Einmal erhielten die Probanden 50 mg (S)-Ketamin OTF und einmal zwei 50 mg (S)-Ketamin OTF (Gesamtdosis damit 100 mg) in zufälliger Reihenfolge. 15 Probanden erhielten die OTF sublingual, 5 weitere Probanden erhielten die OTF bukkal.

### Durchführung der Studie:

Die Probanden erhielten während einer Ausführung entweder ein einzelnes 50 mg (S)-Ketamin-OTF sublingual (n=15) oder bukkal (n=5). Bei einer weiteren Ausführung erhielten die Probanden gleichzeitig zwei 50 mg (S)-Ketamin-OTF sublingual (n=15) oder bukkal (n=5). Sechs Stunden nach der OTF-Verabreichung wurde den Probanden eine niedrige (S)-Ketamin-Dosis (20 mg) intravenös verabreicht. Zwischen den Studientagen lag eine Auswaschphase von mindestens 2 Tagen.

### Blutentnahme:

Zur Blutentnahme wurde ein arterieller Zugang in die linke oder rechte Radial- oder Brachialarterie gelegt. Blutproben (4 ml) wurden zu den folgenden Zeitpunkten nach der OTF-Verabreichung (t = 0 min) entnommen: 0, 5, 10, 20, 40, 60, 90, 120, 180, 240, 300, 360 min und zu den folgenden Zeitpunkten nach Beginn der intravenösen Verabreichung: 2, 4, 10, 15, 20, 30, 40, 60, 75, 90 und 120 min.

### Schmerz-Tests:

Drei Schmerztests wurden in Abständen von 10 bis 20 Minuten durchgeführt (in Abständen von 10 bis 60 Minuten (0 - 10 - 20 - 30 - 40 - 60 - 80 - 100 - 120 - 150 - 180 - 240 - 300 - 360 Minuten)
a) Druckschmerz,
b) elektrischer Schmerz und
c) hitzeinduzierter Schmerz.

a) Mit dem Wagner Instruments FDN 200 Algometer bewerteten die Probanden ihre Druckschmerzschwelle als Reaktion auf einen zunehmenden Druckreiz.
b) Unter Verwendung eines transkutanen elektrischen Schmerzmodells bewertete der Proband die Schmerzschwelle bei einer festen Stimulation der Haut, so dass der Schmerzwert 7 bis 8 betrug.
c) Unter Verwendung des Medoc Pathway Systems wurde ein fester Hitzereiz auf die Haut ausgeübt, der einen Schmerzwert von 7 bis 8 verursachte.

Alle Schmerzbewertungen wurden anhand einer 11-stufigen Likert-Skala (Verbal Rating Scale, VRS) vorgenommen, die von 0 (kein Schmerz) bis 10 (maximal vorstellbarer Schmerz) reichte.

### Qualitative Ergebnisse:

Der Geschmack der Filmformulierungen wurde von den Probanden als akzeptabel beschrieben. Es wurden keine sicherheitsrelevanten Ereignisse berichtet. Die sublinguale als auch bukkale Gabe wurde nicht als problematisch empfunden.

### Plasmakonzentrationen:

Die Plasmakonzentration wurden mittels Liquid-Chromatography gekoppelt mit QTOF-MS als Detektionstechnik gemessen. Die untere Nachweisgrenze betrug 6 ng/mL, 6 ng/mL und 4 ng/mL für jeweils (S)-Ketamin, (S)-Norketamin und (S)-Hydroxynorketamin. Die obere Nachweisgrenze war 1000, 500 und 200 ng/mL für (S)-Ketamin, (S)-Norketamin und (S)-Hydroxynorketamin.

### Fragebögen:

Unmittelbar vor dem Schmerztest wurden zwei Fragebögen im Abstand von 30 Minuten ausgefüllt, um die Wirkung der medikamentösen Behandlung auf psychische und psychomimetische Nebenwirkungen zu beurteilen (0 - 30 - 60 - 90 - usw. von 0 bis 360 Minuten).
1. Bowdle-Fragebogen: Aus dem Bowdle-Fragebogen (Bowdle et al "Psychedelic effects of ketamine in healthy volunteers: relationship to steady-state plasma concentrations" Anesthesiology 1998 Jan; 88(1): 82-8) lassen sich drei Faktoren psychedelischer Wirkungen ableiten: Drogenrausch, innere Wahrnehmung und äußere Wahrnehmung.
2. Bond und Lader Fragebogen: Die Bond und Lader Skalen werden aus sechzehn 100 mm visuellen Analogskalen berechnet. Die Endpunkte sind auf antonymische Wortpaare gesetzt, wie z.B. 'wach-schläfrig', 'gut koordiniert-ungeschickt', 'geistig langsam-schnell-aufmerksam' und 'inkompetent-professionell'.

### Ergebnisse:

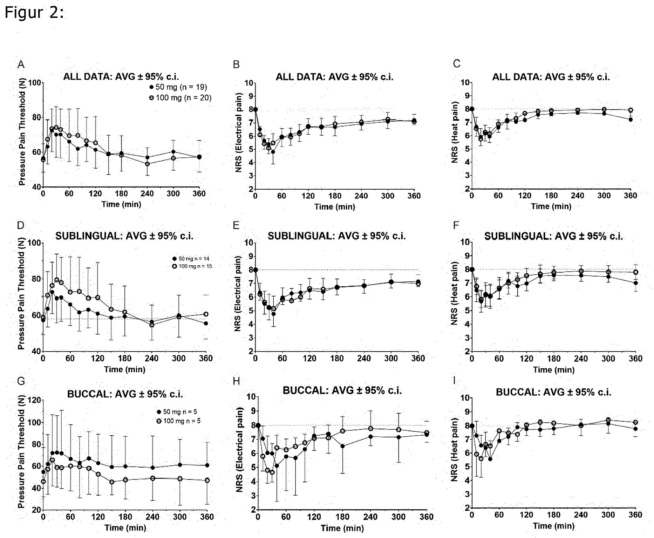

Die Ergebnisse zeigen einen schnell einsetzenden, potenten und langanhaltenden Schmerznachlass (siehe Figur 2). Es wurde keine Dosisabhängigkeit bei der Schmerzlinderung für die OTF Formulierung beobachtet. Dies ist vermutlich auf die hohe Konzentration des Metaboliten Norketamin zurückzuführen, der einen antianalgetischen Effekt bewirkt (siehe Olofsen et al "Estimation of contribution of norketamine to ketamine-induced acute pain relief and neurocognitive impairment in healthy volunteers" Anesthesiology 2012; 117; Addendum 5).

Es wurde ein nicht-linearer dosisabhängiger Anstieg der Konzentration von Ketamin und der Metabolite (S)-Norketamin und (S)-Hydroxynorketamin für die OTF Formulierung beobachtet.

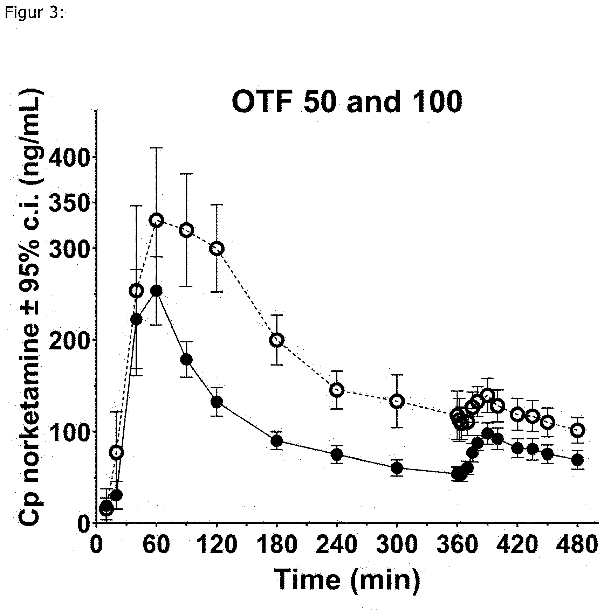

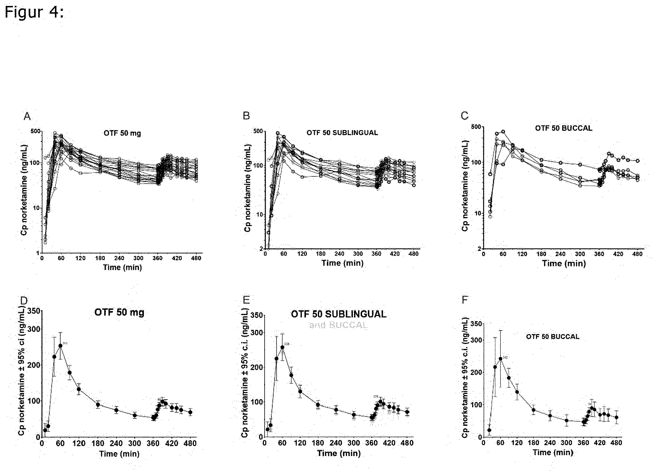

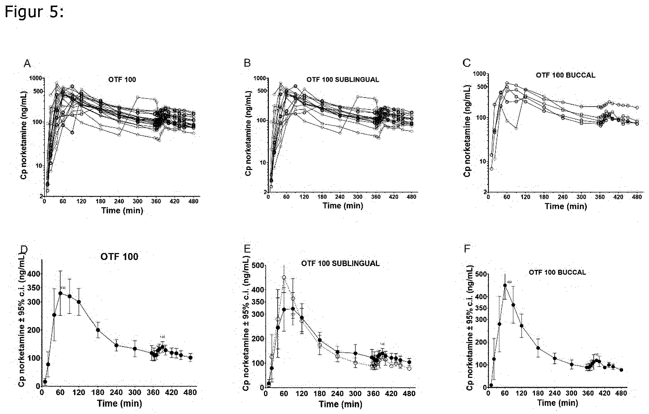

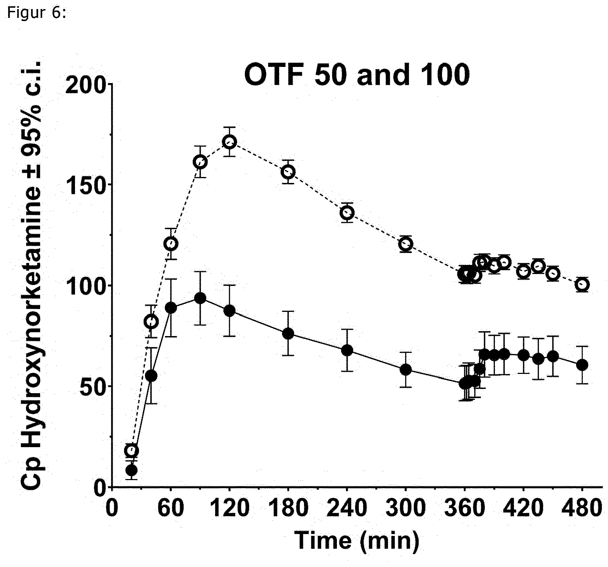

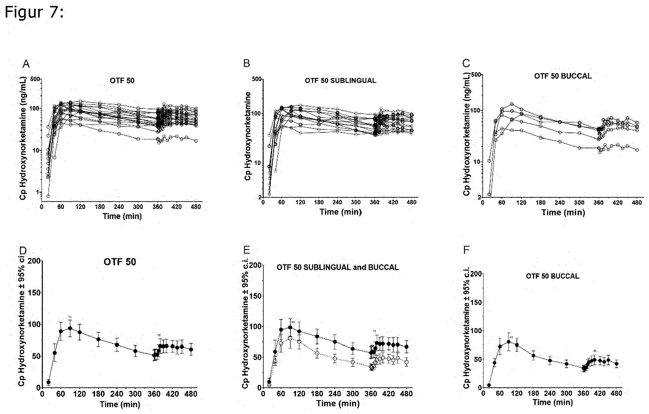

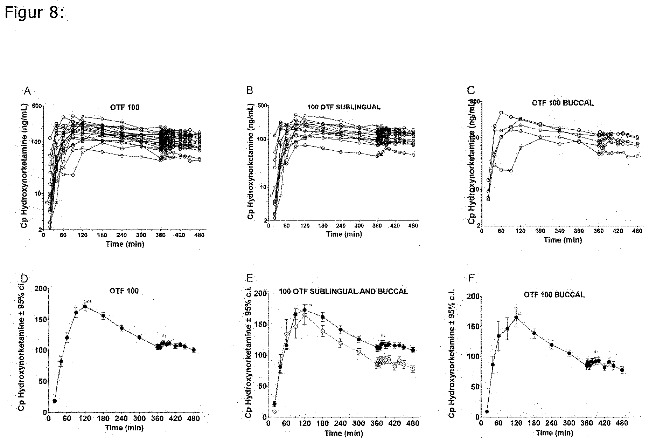

Die Plasmaspiegel der Metabolite (S)-Norketamin (siehe Figuren 3, 4 und 5) und (S)-Hydroxynorketamin (Figuren 6, 7 und 8) sind nach Gabe eines OTFs höher (siehe auch nachfolgende Tabelle 7) als im Vergleich zur intravenösen Verabreichung von 20 mg (S)-Ketamin.

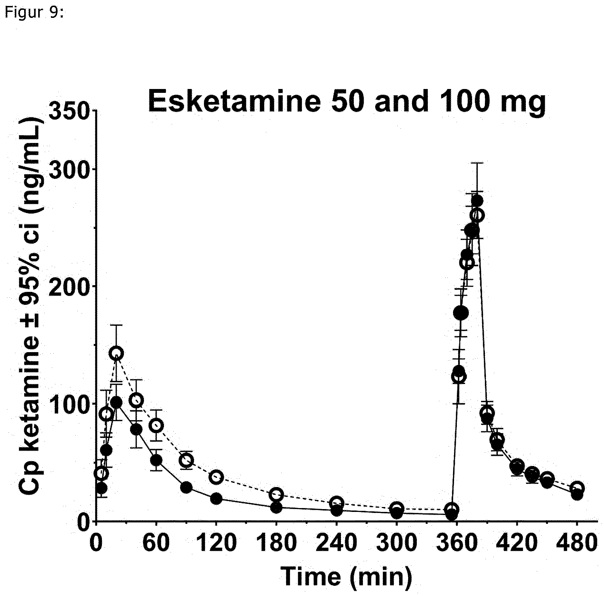

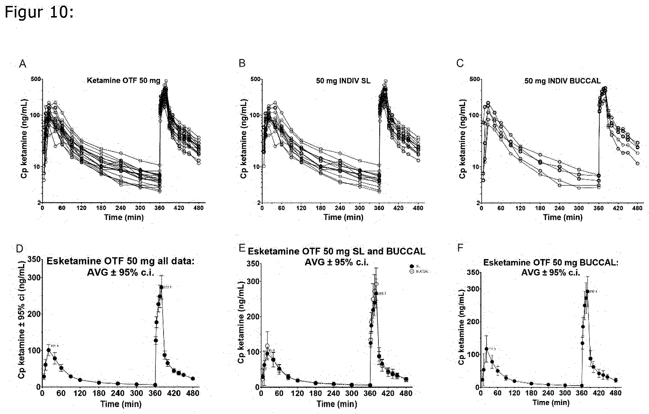

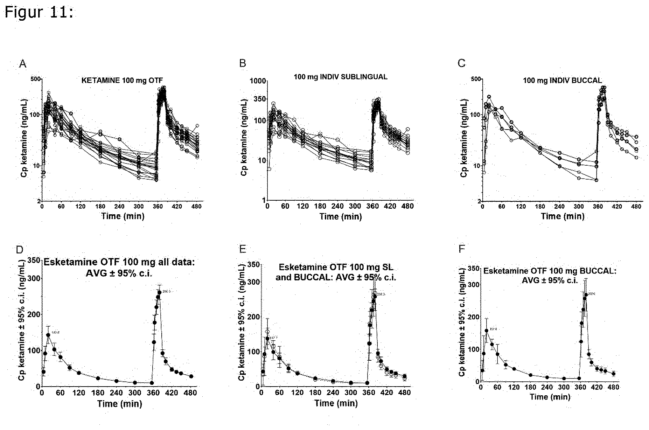

Die Plasmaspiegel an Ketamin sind bei intravenöser Gabe höher als nach Gabe eines OTFs (siehe Figuren 9, 10, 11 und die nachfolgende Tabelle 7).

Die untersuchte Filmformulierung ist für die Behandlung von Schmerzen geeignet.

**Tabelle 7: Cmax Plasma Konzentrationen (ng/ml)**

| | Alle Daten 50 mg | Alle Daten 2x50 mg | Sublingual 50 mg | Sublingual 2x50 mg | Buccal 50 mg | Buccal 2x50 mg |
|---|---|---|---|---|---|---|
| (S)-Ketamin | 101 | 143 | 95 | 138 | 117 | 158 |
| (S)-Norketamin | 254 | 330 | 258 | 323 | 242 | 450 |
| (S)-Hydroxynorketamin | 94 | 171 | 98 | 173 | 81 | 165 |

Nach der Verabreichung eines Films liegen die zu erzielenden Plasmawerte bevorzugt in den folgenden Bereichen: cmax ((S)-Ketamin) = 50-200 ng/mL; cmax (S-Norketamin) = 200-400 ng/mL; cmax (S-Hydroxynorketamin) = 50-150 ng/mL.

Werden zwei Filme verabreicht liegen die zu erzielenden Plasmawerte bevorzugt in den folgenden Bereichen: cmax ((S)-Ketamin) = 100-200 ng/mL; cmax (S-Norketamin) = 300-500 ng/mL; cmax (S-Hydroxynorketamin) = 100-250 ng/mL.

### Orale Bioverfügbarkeit

Die orale Bioverfügbarkeit für das (S)-Ketamin OTF beträgt 26,3 % ± 1,0%.

Die orale Bioverfügbarkeit von 50 mg und 100 mg (S)-Ketamin OTF unterscheidet sich um etwa 20 % (F1 50 mg = 29 %, F1 100 mg = 23 %), was jedoch nicht das Signifikanzniveau erreicht (p » 0,01).

### Tmax, AUC Daten berechnet mit Hilfe eines PK Models

Die Datenanalyse der gemessenen Plasmakonzentrationen von (S)-Ketamin und seiner Metabolite zur Ermittlung eines pharmakokinetischen Models erfolgte mit NONMEM Version 7.5.0 (ICON Development Solutions, Hannover, MD, USA).

FOCI-I (first-order conditional estimation with interaction) wurde zur Berechnung der pharmakokinetischen Model Parameter eingesetzt. Basierend auf dem erstellten pharmakokinetischen Model wurden TMax Daten und AUC Daten von (S)-Ketamin und seinen Metaboliten berechnet und sind in Tabelle 8 aufgeführt.

**Tabelle 8:**

| | 50 mg (S)-Ketamin OTF | 100 mg (S)-Ketamin OTF |
|---|---|---|
| | (S)-Ketamin | |
| Tmax (min) | 18,8 (16,6 - 21,2) | 19,1 (17,1 - 21,2) |
| AUC (0-6) (ng/mL.min) | 8,363 (7,263 - 9,464) | 13.347 (11,933 - 14,760) |

| | (S)-Norketamin | |
|---|---|---|
| Tmax (min) | 61 (53-68) | 78 (66-91) |
| AUC (0-6) (ng/mL.min) | 38,497 (34,131 - 42,863) | 67,959 (60,045 - 75,872) |

| | (S)-Hydroxynorketamin | |
|---|---|---|
| Tmax (min) | 81 (69-92) | 109 (89 - 130) |
| AUC (0-6) (ng/mL.min) | 24,087 (20,694 - 27,480) | 44,972 (38,563 - 51,382) |

Weitere Ergebnisse der Studie lauten wie folgt:
1. Die erfindungsgemäße OTF Formulierung ist in allen drei Schmerzmodalitäten analgetisch, unabhängig von der Lokalisation.
2. In keinem der angewandten Schmerztests zeigt sich eine klare Dosis-Wirkungs-Beziehung.
3. In dem elektrischen und thermischen Schmerztest ist die analgetische Wirkung langanhaltend und reicht von 2 bis 6 Stunden, insbesondere nach sublingualer Verabreichung des OTF.
4. Es gibt keine offensichtlichen Unterschiede in den Daten, die während und nach sublingualer und bukkaler Verabreichung erhalten wurden.
5. Die psychedelischen Effekte sind für das verabreichte OTF als sehr mild anzusehen.
6. Achtzehn (18) Probanden berichteten über mindestens ein unerwünschtes Ereignis. Insgesamt gab es 33 unerwünschte Ereignisse. Keines davon war ein schwerwiegendes unerwünschtes Ereignis (siehe für die Prävalenz der Ereignisse die nachfolgende Tabelle 9). Die unerwünschten Ereignisse wurden primär für die intravenöse Verabreichung von 20 mg (S)-Ketamin beobachtet.

**Tabelle 9:**

| Nebenwirkung | 50 mg (S)-Ketamin | 100 mg (S)-Ketamin | 20 mg (S)-Ketamin IV |
|---|---|---|---|
| Unscharfe Sicht | 1 | --- | --- |
| Gefühl der Trunkenheit | 2 | --- | --- |
| Bradykinesie | 1 | 1 | --- |
| Pfeifendes Ohrgeräusch | --- | --- | 1 |
| Schwindel | 1 | 3 | 4 |
| Schläfrigkeit | --- | --- | 3 |
| Übelkeit | 1 | 1 | 2 |
| Kopfschmerz | 1 | 2 | 3 |
| Taubheit der Zunge | --- | 2 | ---. |
| Bluthochdruck (SBP>180mm Hg) | --- | --- | 2 |
| Schwitzen | --- | --- | 1 |
| Trockene Augen | --- | --- | 1 |
| Total | 7 | 9 | 17 |

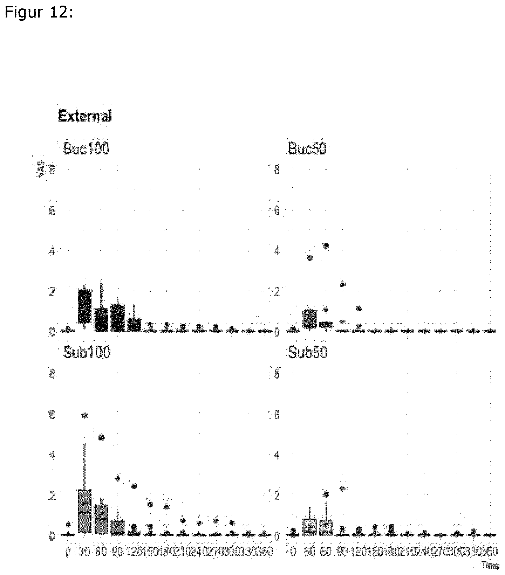

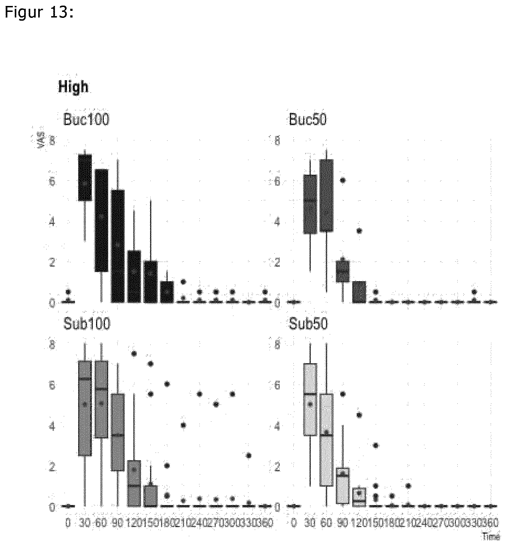

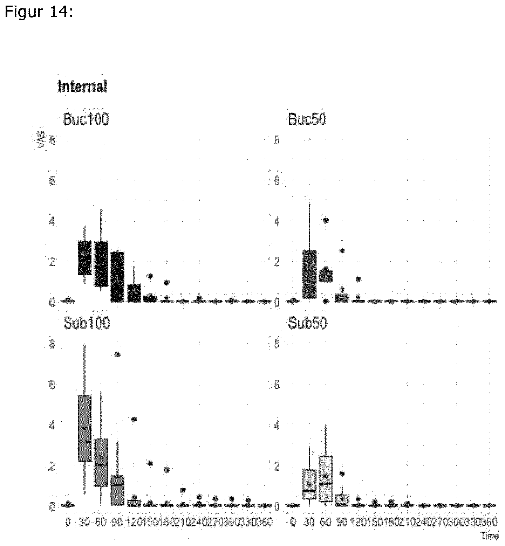

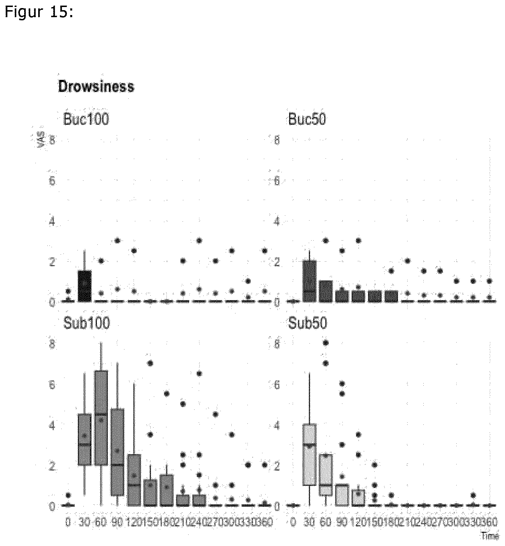

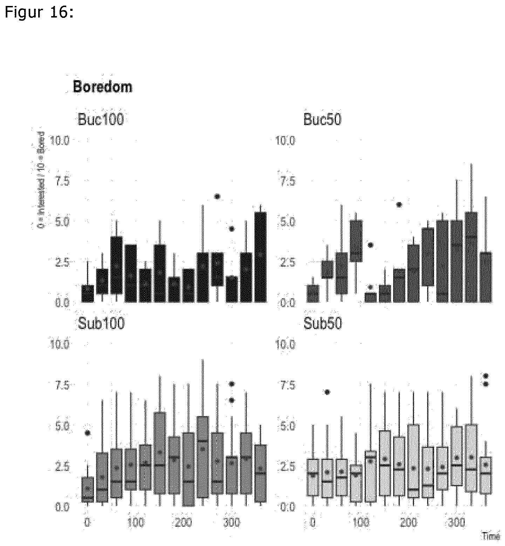

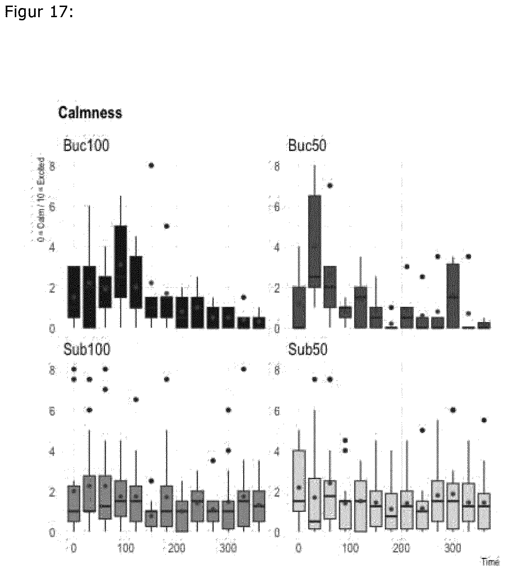

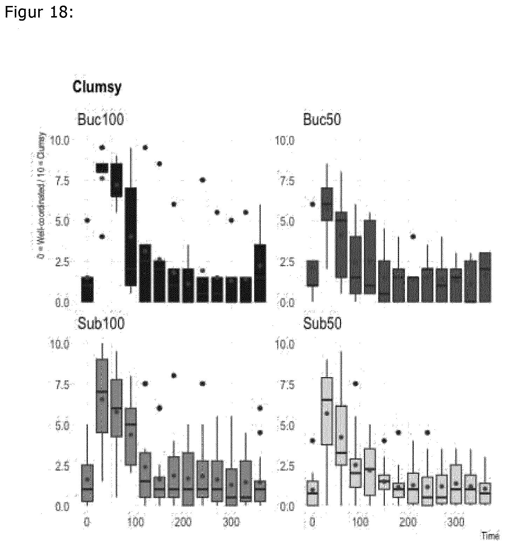

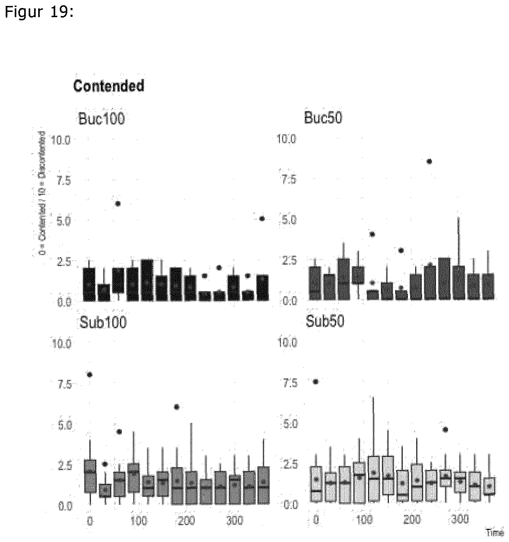

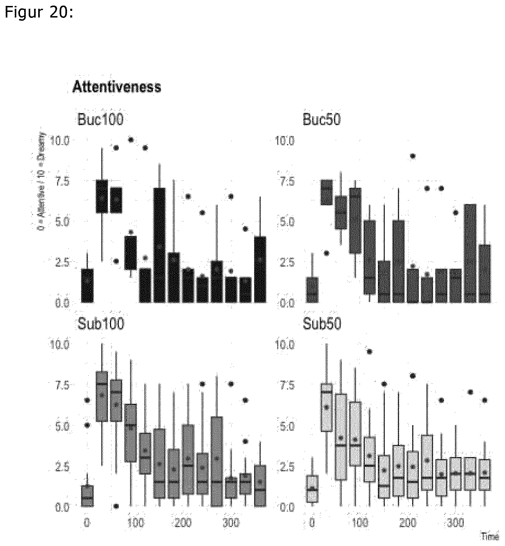

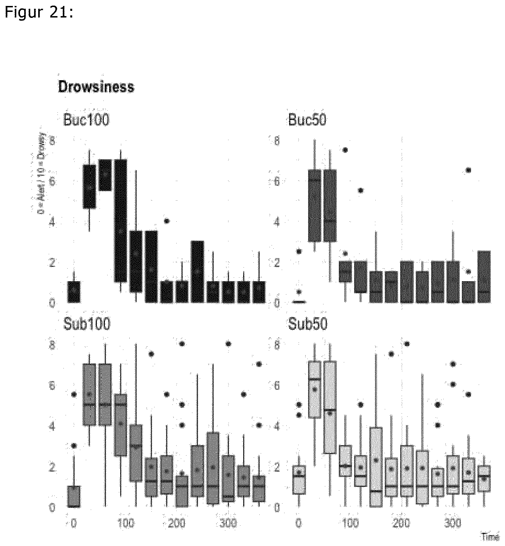

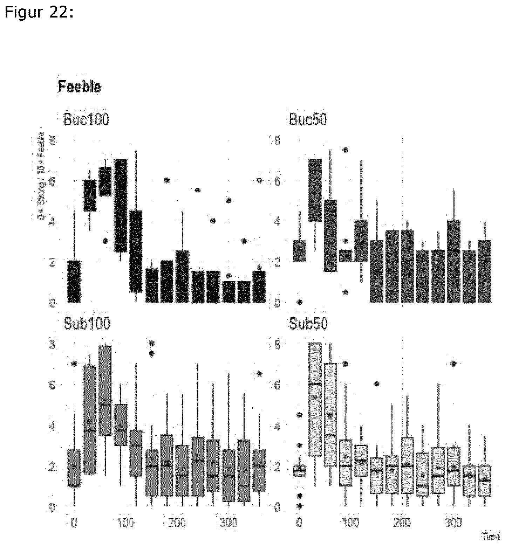

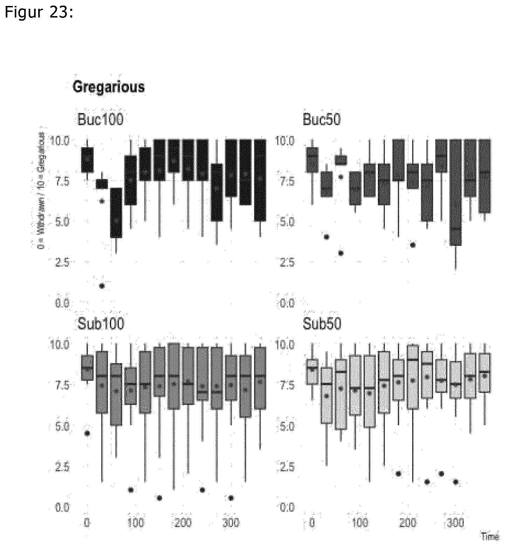

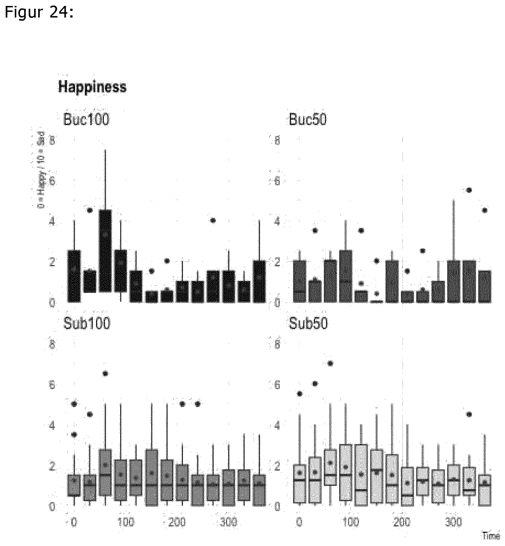

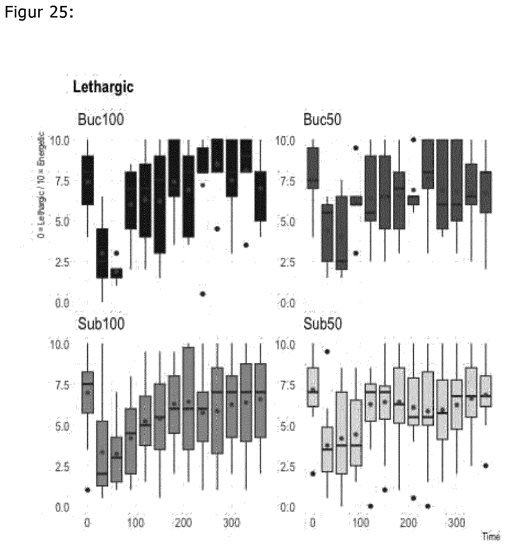

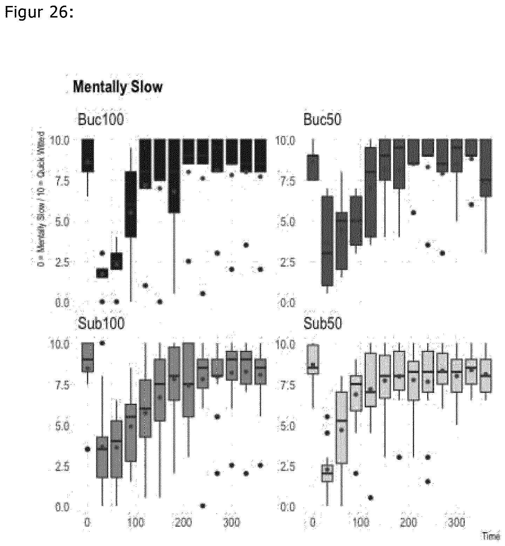

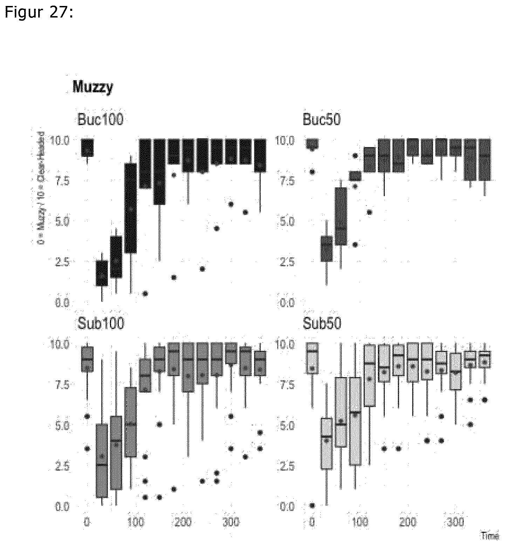

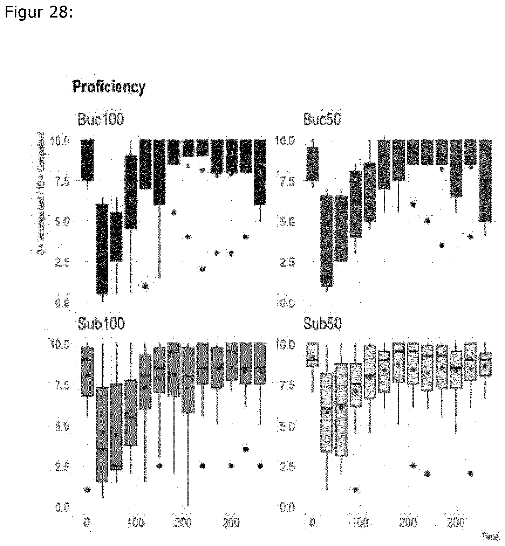

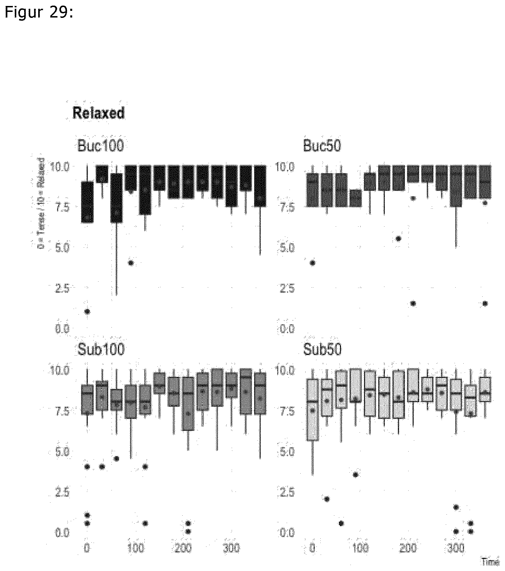

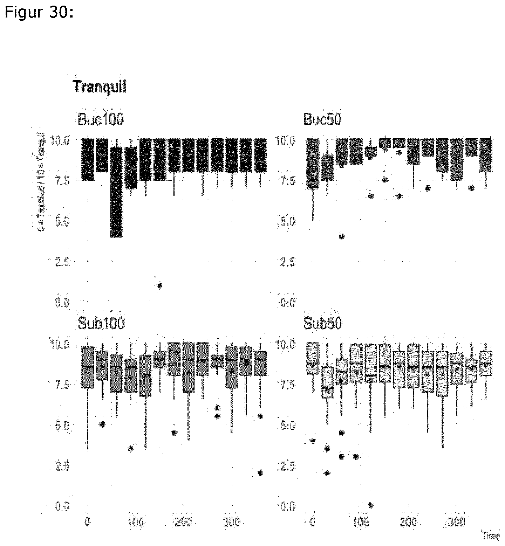

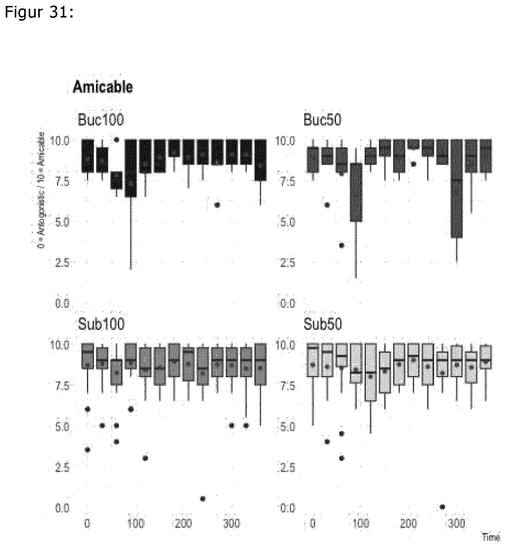

Die Daten zu den beobachteten psychedelischen Effekten (psychische und psychomimetische Nebenwirkungen) nach dem Bowdle Fragebogen sind in den Figuren 12 bis 15 und nach dem Bond und Lader Fragebogen in den Figuren 16 bis 31 zusammengefasst.

### Beispiel 5:

Es wurden die Wirkstoffe (R)-Ketamin und (S)-Ketamin verglichen.

Dazu wurden orale Dünnfilme der Zusammensetzung gemäß Tabelle 10 hergestellt.

**Tabelle 10:**

| | | |
|---|---|---|
| **Material** | **15 [Gew.-%]** | **16 [Gew.-%]** |

| (S)-Ketamin HCl | 60,0 | - |
|---|---|---|
| R-Ketamin HCl | | 60,0 |
| PVA 4-88 | - | - |
| PVA 40-88 | 10,0 | 10,0 |
| Kollicoat IR | 20,1 | 20,1 |
| Saccharin Na | 1,0 | 1,0 |
| Sucralose | 2,0 | 2,0 |
| Glycerol | 3,5 | 3,5 |
| Cherry flavor M55394 | 3,0 | 3,0 |
| FD&C Red No, 40 | 0,4 | 0,4 |
| **Lösungsmittel** | Aq. Pur. | Aq. Pur. |
| **Flächengewicht** | 215,73 g/m² | 221,4 g/m² |

Im Folgenden wurde der Wirkstoffflux im in-vitro Modell bestimmt.

Der Wirkstoffflux wurde im Rahmen einer typischen in vitro-Permeation mittels Franz-Diffusionszellen (Volumen 10 mL) bei 37°C durchgeführt. Zu vorbestimmten Wechselzeiten wurde das jeweils verwendete Akzeptormedium komplett durch ein Neues ausgetauscht und der Gehalt an permeierter Wirkstoffmenge in diesen Akzeptorlösungen mittels HPLC bestimmt.

Als Akzeptormedium wurde Phosphatpuffer (pH 7,4) eingesetzt.

Als Hautmodell wurde dermatomisierte Haut aus der Speiseröhre eines Schweins mit einer Schichtdicke von 400µm verwendet.

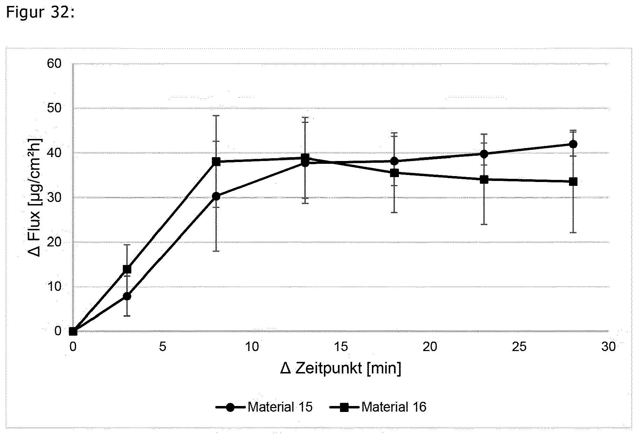

Die Ergebnisse der Permationsstudie sind in Figur 32 dargestellt.

## Patentansprüche

1. Oraler Dünnfilm, umfassend mindestens eine Matrixschicht, wobei die mindestens eine Matrixschicht mindestens einen pharmazeutisch aktiven Wirkstoff, mindestens einen Polyvinylalkohol und mindestens ein Polyvinylalkohol-Polyethylenglycol-Pfropfcopolymer umfasst, wobei der mindestens eine pharmazeutisch aktive Wirkstoff Ketamin oder ein pharmazeutisch akzeptables Salz davon umfasst.

2. Oraler Dünnfilm gemäß Anspruch 1, wobei der mindestens eine pharmazeutisch aktiven Wirkstoff (S)-Ketamin oder ein pharmazeutisch akzeptables Salz davon umfasst.

3. Oraler Dünnfilm gemäß irgendeinem der vorhergehenden Ansprüche, wobei der mindestens eine pharmazeutisch aktive Wirkstoff in einer Menge von 45 bis 70 Gew.-%, vorzugsweise von 50 bis 65 Gew.-%, bezogen auf das Gesamtgewicht der Matrixschicht, in der Matrixschicht vorhanden ist.

4. Oraler Dünnfilm gemäß irgendeinem der vorhergehenden Ansprüche, wobei der mindestens eine Polyvinylalkohol einen Polyvinylalkohol mit einem mittleren Molekulargewicht von etwa 25.000 bis etwa 250.000 g/mol umfasst.

5. Oraler Dünnfilm gemäß irgendeinem der vorhergehenden Ansprüche, wobei der mindestens eine Polyvinylalkohol einen Polyvinylalkohol mit einem mittleren Molekulargewicht von etwa 25.000 bis etwa 35.000 g/mol und/oder einen Polyvinylalkohol mit einem mittleren Molekulargewicht von etwa 200.000 bis 210.000 g/mol umfasst.

6. Oraler Dünnfilm gemäß irgendeinem der vorhergehenden Ansprüche, wobei das mindestens eine Polyvinylalkohol-Polyethylenglycol-Pfropfcopolymer eine Polyethylenglycol-Hauptkette aufweist, auf die Polyvinylalkohol-Einheiten aufgepfropft sind.

7. Oraler Dünnfilm gemäß irgendeinem der vorhergehenden Ansprüche, wobei das mindestens eine Polyvinylalkohol-Polyethylenglycol-Pfropfcopolymer eine Polyethylenglycol-Hauptkette aufweist, auf die Polyvinylalkohol-Einheiten aufgepfropft sind, wobei das molare Verhältnis von Polyethylenglycol zu Polyvinylalkohol 1:3 beträgt.

8. Oraler Dünnfilm gemäß irgendeinem der vorhergehenden Ansprüche, wobei das mindestens eine Polyvinylalkohol-Polyethylenglycol-Pfropfcopolymer eine Polyethylenglycol-Hauptkette aufweist, auf die Polyvinylalkohol-Einheiten aufgepfropft sind, wobei das Polyvinylalkohol-Polyethylenglycol-Pfropfcopolymer ein mittleres Molekulargewicht im Bereich von 40.000 bis 50.000 g/mol, bevorzugt von etwa 45000 g/mol, aufweist.

9. Oraler Dünnfilm gemäß irgendeinem der vorhergehenden Ansprüche, wobei der mindestens eine Polyvinylalkohol in einer Menge von 5 bis 40 Gew.-%, vorzugsweise von 5 bis 10 Gew.-%, bezogen auf das Gesamtgewicht der Matrixschicht, in der Matrixschicht vorhanden ist.

10. Oraler Dünnfilm gemäß irgendeinem der vorhergehenden Ansprüche, wobei das mindestens eine Polyvinylalkohol-Polyethylenglycol-Pfropfcopolymer in einer Menge von 15 bis 45 Gew.-%, vorzugsweise von 17 bis 40 Gew.-%, bezogen auf das Gesamtgewicht der Matrixschicht, in der Matrixschicht vorhanden ist.

11. Oraler Dünnfilm gemäß irgendeinem der vorhergehenden Ansprüche, wobei der orale Dünnfilm ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

12. Oraler Dünnfilm gemäß irgendeinem der vorhergehenden Ansprüche, wobei das Flächengewicht des oralen Dünnfilms etwa 50 bis 300 g/m² beträgt.

13. Oraler Dünnfilm gemäß irgendeinem der vorhergehenden Ansprüche, wobei der mindestens eine pharmazeutisch aktiven Wirkstoff Ketamin als freie Base in einer Gesamtmenge von 25 bis 150 mg, vorzugsweise von etwa 50 bis 150 mg, umfasst.

14. Oraler Dünnfilm gemäß irgendeinem der vorhergehenden Ansprüche, wobei mindestens 40 % oder mindestens 50 % des mindestens einen pharmazeutisch aktiven Wirkstoffs innerhalb der ersten Minute nach Applikation freigesetzt werden und/oder wobei mindestens 75 %, mindestens 80% oder mindestens 85% des mindestens einen pharmazeutisch aktiven Wirkstoffs innerhalb der ersten zwei Minuten nach Applikation freigesetzt werden.

15. Oraler Dünnfilm gemäß irgendeinem der vorhergehenden Ansprüche, wobei die Durchstoßfestigkeit mindestens 0,15 N/mm², vorzugsweise mindestens 0,18 N/mm² und besonders bevorzugt mindestens 0,20 N/mm², bei einem Flächengewicht von 150 bis 250 g/m², bevorzugt von 180 bis 220 g/m², beträgt.

16. Oraler Dünnfilm gemäß irgendeinem der vorhergehenden Ansprüche, wobei die Matrixschicht 60 Gew.-% (S)-Ketamin HCl, 10 Gew.- eines Polyvinylalkohols mit einem mittleren Molekulargewicht von etwa 200.000 bis 210.000 g/mol, bevorzugt von 205.000 g/mol, und 20,1 Gew.-% eines Polyvinylalkohol-Polyethylenglycol-Pfropfcopolymers, wobei das Polyvinylalkohol-Polyethylenglycol-Pfropfcopolymer eine Polyethylenglycol-Hauptkette aufweist, auf die Polyvinylalkohol-Einheiten aufgepfropft sind, und wobei das Polyvinylalkohol-Polyethylenglycol-Pfropfcopolymer ein mittleres Molekulargewicht im Bereich von 40.000 bis 50.000 g/mol, bevorzugt von etwa 45000 g/mol, aufweist, umfasst.

17. Verfahren zur Herstellung eines oralen Dünnfilms gemäß irgendeinem der Ansprüche 1 bis 16, umfassend die Schritte:
a) Herstellen einer Lösung, Dispersion oder Schmelze, umfassend den mindestens einen pharmazeutisch aktiven Wirkstoff, den mindestens einen Polyvinylalkohol und das mindestens eine Polyvinylalkohol-Polyethylenglycol-Pfropfcopolymer;
a1) optional Aufschäumen der Lösung, Dispersion oder Schmelze aus Schritt a) durch Eintragen eines Gases oder Gasgemisches, durch chemische Gaserzeugung oder durch Entspannen eines gelösten Gases,
b) Ausstreichen der Lösung, Dispersion oder Schmelze aus Schritt a) bzw. der optional aufgeschäumten Lösung, Dispersion oder Schmelze aus Schritt a1.

18. Oraler Dünnfilm gemäß irgendeinem der Ansprüche 1 bis 16 oder erhältlich nach dem Verfahren gemäß Anspruch 17 zur Verwendung als Arzneimittel.

19. Oraler Dünnfilm gemäß irgendeinem der Ansprüche 1 bis 16 oder erhältlich nach dem Verfahren gemäß Anspruch 17 zur Verwendung in der Behandlung von Schmerzen und/oder Depressionen.

## Claims

1. An oral thin film comprising at least one matrix layer, wherein the at least one matrix layer comprises at least one pharmaceutically active agent, at least one polyvinyl alcohol, and at least one polyvinyl alcohol-polyethylene glycol graft copolymer, wherein the at least one pharmaceutically active agent comprises ketamine or a pharmaceutically acceptable salt thereof.

2. The oral thin film according to claim 1, wherein the at least one pharmaceutically active agent comprises (S)-ketamine or a pharmaceutically acceptable salt thereof.

3. The oral thin film according to any one of the preceding claims, wherein the at least one pharmaceutically active agent is provided in the matrix layer in an amount of 45 to 70 wt.%, preferably of 50 to 65 wt.%, in relation to the total weight of the matrix layer.

4. The oral thin film according to any one of the preceding claims, wherein the at least one polyvinyl alcohol comprises a polyvinyl alcohol with a mean molecular weight of approximately 25,000 to approximately 250,000 g/mol.

5. The oral thin film according to any one of the preceding claims, wherein the at least one polyvinyl alcohol comprises a polyvinyl alcohol with a mean molecular weight of approximately 25,000 to approximately 35,000 g/mol and/or a polyvinyl alcohol with a mean molecular weight of approximately 200,000 to 210,000 g/mol.

6. The oral thin film according to any one of the preceding claims, wherein the at least one polyvinyl alcohol-polyethylene glycol graft copolymer has a polyethylene glycol main chain onto which there are grafted polyvinyl alcohol units.

7. The oral thin film according to any one of the preceding claims, wherein the at least one polyvinyl alcohol-polyethylene glycol graft copolymer has a polyethylene glycol main chain onto which there are grafted polyvinyl alcohol units, wherein the molar ratio of polyethylene glycol to polyvinyl alcohol is 1:3.

8. The oral thin film according to any one of the preceding claims, wherein the at least one polyvinyl alcohol-polyethylene glycol graft copolymer has a polyethylene glycol main chain onto which there are grafted polyvinyl alcohol units, wherein the polyvinyl alcohol-polyethylene glycol graft copolymer has a mean molecular weight in the range of 40,000 to 50,000 g/mol, preferably of approximately 45,000 g/mol.

9. The oral thin film according to any one of the preceding claims, wherein the at least one polyvinyl alcohol is provided in the matrix layer in an amount of 5 to 40 wt.%, preferably of 5 to 10 wt.%, in relation to the total weight of the matrix layer.

10. The oral thin film according to any one of the preceding claims, wherein the at least one polyvinyl alcohol-polyethylene glycol graft copolymer is provided in the matrix layer in an amount of 15 to 45 wt.%, preferably of 17 to 40 wt.%, in relation to the total weight of the matrix layer.

11. The oral thin film according to any one of the preceding claims, wherein the oral thin film further comprises at least one auxiliary substance selected from the group comprising colouring agents, flavourings, sweeteners, plasticisers, taste-masking agents, emulsifiers, enhancers, pH regulators, humectants, preservatives and/or antioxidants.

12. The oral thin film according to any one of the preceding claims, wherein the area density of the oral thin film is approximately 50 to 300 g/m².

13. The oral thin film according to any one of the preceding claims, wherein the at least one pharmaceutically active agent comprises ketamine as a free base in a total amount of 25 to 150 mg, preferably of approximately 50 to 150 mg.

14. The oral thin film according to any one of the preceding claims, wherein at least 40% or at least 50% of the at least one pharmaceutically active agent are released within the first minute following application, and/or wherein at least 75%, at least 80% or at least 85% of the at least one pharmaceutically active agent are released within the first two minutes following application.

15. The oral thin film according to any one of the preceding claims, wherein the puncture strength is at least 0.15 N/mm², preferably at least 0.18 N/mm² and especially preferably at least 0.20 N/mm², with an areal density of 150 to 250 g/m², preferably of 180 to 220 g/m².

16. The oral thin film according to any one of the preceding claims, wherein the matrix layer comprises 60 wt.% of (S)-ketamine HCl, 10 wt.% of a polyvinyl alcohol with a mean molecular weight of approximately 200,000 to 210,000 g/mol, preferably of 205,000 g/mol, and 20.1 wt.% of a polyvinyl alcohol-polyethylene glycol graft copolymer, wherein the polyvinyl alcohol-polyethylene glycol graft copolymer has a polyethylene glycol main chain onto which there are grafted polyvinyl alcohol units, and wherein the polyvinyl alcohol-polyethylene glycol graft copolymer has a mean molecular weight in the range of 40,000 to 50,000 g/mol, preferably of approximately 45,000 g/mol.

17. A method for producing an oral thin film according to any one of claims 1 to 16, comprising the steps of:
a) producing a solution, dispersion or melt comprising the at least one pharmaceutically active agent, the at least one polyvinyl alcohol and the at least one polyvinyl alcohol-polyethylene glycol graft copolymer;
a1) optionally foaming the solution, dispersion or melt from step a) by introducing a gas or gas mixture, by chemical gas generation or by expansion of a dissolved gas,
b) spreading the solution, dispersion or melt from step a) or the optionally foamed solution, dispersion or melt from step a1.

18. The oral thin film according to any one of claims 1 to 16 or obtainable by the method according to claim 17 for use as a medicament.

19. The oral thin film according to any one of claims 1 to 16 or obtainable by the method according to claim 17 for use in the treatment of pain and/or depression.

## Revendications

1. Film mince d'hygiène bucco-dentaire, comprenant au moins une couche de matrice, dans lequel l'au moins une couche de matrice comprend au moins un principe pharmaceutiquement actif, au moins un alcool polyvinylique et au moins un copolymère de greffage d'alcool polyvinylique-polyéthylène glycol, dans lequel l'au moins un principe pharmaceutiquement actif comprend de la kétamine ou un sel pharmaceutiquement acceptable de celle-ci.

2. Film mince d'hygiène bucco-dentaire selon la revendication 1, dans lequel l'au moins un principe pharmaceutiquement actif comprend de la S-kétamine ou un sel pharmaceutiquement acceptable de celle-ci.

3. Film mince d'hygiène bucco-dentaire selon l'une quelconque des revendications précédentes, dans lequel l'au moins un principe pharmaceutiquement actif est présent dans la couche de matrice en une quantité de 45 à 70 % en poids, de préférence de 50 à 65 % en poids, par rapport au poids total de la couche de matrice.

4. Film mince d'hygiène bucco-dentaire selon l'une quelconque des revendications précédentes, dans lequel l'au moins un alcool polyvinylique comprend un alcool polyvinylique avec un poids moléculaire moyen d'environ 25 000 à environ 250 000 g/mol.

5. Film mince d'hygiène bucco-dentaire selon l'une quelconque des revendications précédentes, dans lequel l'au moins un alcool polyvinylique comprend un alcool polyvinylique avec un poids moléculaire moyen d'environ 25 000 à environ 35 000 g/mol et/ou un alcool polyvinylique avec un poids moléculaire moyen d'environ 200 000 à 210 000 g/mol.

6. Film mince d'hygiène bucco-dentaire selon l'une quelconque des revendications précédentes, dans lequel l'au moins un copolymère de greffage d'alcool polyvinylique-polyéthylène glycol présente une chaîne principale de polyéthylène glycol, sur laquelle les motifs d'alcool polyvinylique sont greffés.

7. Film mince d'hygiène bucco-dentaire selon l'une quelconque des revendications précédentes, dans lequel l'au moins un copolymère de greffage d'alcool polyvinylique-polyéthylène glycol présente une chaîne principale de polyéthylène glycol, sur laquelle des motifs d'alcool polyvinylique sont greffés, dans lequel le rapport molaire du polyéthylène glycol par rapport à l'alcool polyvinylique est de 1:3.

8. Film mince d'hygiène bucco-dentaire selon l'une quelconque des revendications précédentes, dans lequel l'au moins un copolymère de greffage d'alcool polyvinylique-polyéthylène glycol présente une chaîne principale de polyéthylène glycol, sur laquelle des motifs d'alcool polyvinylique sont greffés, dans lequel le copolymère de greffage d'alcool polyvinylique-polyéthylène glycol présente un poids moléculaire moyen dans la plage de 40 000 à 50 000 g/mol, de manière préférée d'environ 45 000 g/mol.

9. Film mince d'hygiène bucco-dentaire selon l'une quelconque des revendications précédentes, dans lequel l'au moins un alcool polyvinylique est présent dans la couche de matrice en une quantité de 5 à 40 % en poids, de préférence de 5 à 10 % en poids, par rapport au poids total de la couche de matrice.

10. Film mince d'hygiène bucco-dentaire selon l'une quelconque des revendications précédentes, dans lequel l'au moins un copolymère de greffage d'alcool polyvinylique-polyéthylène glycol est présent dans la couche de matrice en une quantité de 15 à 45 % en poids, de préférence de 17 à 40 % en poids par rapport au poids total de la couche de matrice.

11. Film mince d'hygiène bucco-dentaire selon l'une quelconque des revendications précédentes, dans lequel le film mince d'hygiène bucco-dentaire comprend en outre au moins un adjuvant choisi parmi le groupe comprenant des colorants, des substances aromatiques, des substances édulcorantes, des plastifiants, des agents de masquage du goût, des émulsifiants, des activateurs, des régulateurs de pH, des humectants, des agents de conservation et/ou des anti-oxydants.

12. Film mince d'hygiène bucco-dentaire selon l'une quelconque des revendications précédentes, dans lequel le grammage du film mince d'hygiène bucco-dentaire va d'environ 50 à 300 g/m².

13. Film mince d'hygiène bucco-dentaire selon l'une quelconque des revendications précédentes, dans lequel l'au moins un principe pharmaceutiquement actif comprend de la kétamine en tant que base libre en une quantité totale de 25 à 150 mg, de préférence d'environ 50 à 150 mg.

14. Film mince d'hygiène bucco-dentaire selon l'une quelconque des revendications précédentes, dans lequel au moins 40 % ou au moins 50 % de l'au moins un principe pharmaceutiquement actif sont libérés après application dans la première minute et/ou dans lequel au moins 75 %, au moins 80 % ou au moins 85 % de l'au moins un principe pharmaceutiquement actif sont libérés après application dans les deux premières minutes.

15. Film mince d'hygiène bucco-dentaire selon l'une quelconque des revendications précédentes, dans lequel la résistance à la perforation est d'au moins 0,15 N/mm², de préférence d'au moins 0,18 N/mm² et de manière particulièrement préférée d'au moins 0,20 N/mm², pour un grammage de 150 à 250 g/m², de manière préférée de 180 à 220 g/m².

16. Film mince d'hygiène bucco-dentaire selon l'une quelconque des revendications précédentes, dans lequel la couche de matrice comprend 60 % en poids de (S)-kétamine HCl, 10 % en poids d'un alcool polyvinylique avec un poids moléculaire moyen d'environ 200 000 à 210 000 g/mol, de manière préférée de 205 000 g/mol, et 20,1 % en poids d'un copolymère de greffage d'alcool polyvinylique-polyéthylène glycol, dans lequel le copolymère de greffage d'alcool polyvinylique-polyéthylène glycol présente une chaîne principale de polyéthylène glycol, sur laquelle des motifs d'alcool polyvinylique sont greffés, et dans lequel le copolymère de greffage d'alcool polyvinylique-polyéthylène glycol présente un poids moléculaire moyen dans la plage de 40 000 à 50 000 g/mol, de manière préférée d'environ 45 000 g/mol.

17. Procédé de fabrication d'un film mince d'hygiène bucco-dentaire selon l'une quelconque des revendications précédentes 1 à 16, comprenant les étapes :
a) de fabrication d'une solution, d'une dispersion ou d'une matière fondue, comprenant l'au moins un principe pharmaceutiquement actif, l'au moins un alcool polyvinylique et l'au moins un copolymère de greffage d'alcool polyvinylique-polyéthylène glycol ;
a1) de moussage en option de la solution, de la dispersion ou de la matière fondue provenant de l'étape a) par apport d'un gaz ou d'un mélange de gaz, par production de gaz par voie chimique ou par détente d'un gaz dissous,
b) d'étalement de la solution, de la dispersion ou de la matière fondue provenant de l'étape a) ou de la solution, de la dispersion ou de la matière fondue moussées en option provenant de l'étape a1.

18. Film mince d'hygiène bucco-dentaire selon l'une quelconque des revendications 1 à 16 ou pouvant être obtenu selon le procédé selon la revendication 17 destiné à être utilisé en tant que médicament.

19. Film mince d'hygiène bucco-dentaire selon l'une quelconque des revendications 1 à 16 ou pouvant être obtenu selon le procédé selon la revendication 17, destiné à être utilisé dans le traitement de la douleur et/ou de la dépression.
